(19) Europäisches Patentamt
European Patent Office
Office européen des brevets

(11) **EP 3 271 731 B1**

(12) **EUROPÄISCHE PATENTSCHRIFT**

(45) Veröffentlichungstag und Bekanntmachung des Hinweises auf die Patenterteilung:
**22.05.2019 Patentblatt 2019/21**

(21) Anmeldenummer: **15820531.0**

(22) Anmeldetag: **23.12.2015**

(51) Int Cl.:
*G01N 33/74* [(2006.01)]    *C07K 14/575* [(2006.01)]

(86) Internationale Anmeldenummer:
**PCT/EP2015/081186**

(87) Internationale Veröffentlichungsnummer:
**WO 2016/146221 (22.09.2016 Gazette 2016/38)**

(54) **IN-VITRO-VERFAHREN ZUM NACHWEIS VON MUTIERTEM LEPTIN UND VERWENDUNG EINES NACHWEISREAGENZES**

IN VITRO METHOD FOR DETECTING MUTATED LEPTIN AND USE OF A DETECTION REAGENT

PROCÉDÉ IN VITRO DE DÉTECTION DE LA LEPTINE MUTÉE ET UTILISATION D'UN RÉACTIF DE DÉTECTION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.03.2015 EP 15159303**

(43) Veröffentlichungstag der Anmeldung:
**24.01.2018 Patentblatt 2018/04**

(73) Patentinhaber: **Mediagnost Gesellschaft für Forschung und Herstellung von Diagnostika GmbH 72770 Kusterdingen (DE)**

(72) Erfinder: **FLEHMIG, Bertram 72076 Tübingen (DE)**

(74) Vertreter: **Louis Pöhlau Lohrentz Patentanwälte Postfach 30 55 90014 Nürnberg (DE)**

(56) Entgegenhaltungen:
• N. LAHLOU ET AL: "Soluble leptin receptor in serum of subjects with complete resistance to leptin: relation to fat mass", DIABETES, Bd. 49, Nr. 8, 1. August 2000 (2000-08-01) , Seiten 1347-1352, XP055041870, ISSN: 0012-1797, DOI: 10.2337/diabetes.49.8.1347

• WU ZIDA ET AL: "Quantification of the soluble leptin receptor in human blood by ligand-mediated immunofunctional assay", JOURNAL OF CLINICAL ENDOCRINOLOGY & METABOLISM, Bd. 87, Nr. 6, 1. Juni 2002 (2002-06-01), Seiten 2931-2939, XP002323286,

• MARTIN WABITSCH ET AL: "Biologically Inactive Leptin and Early-Onset Extreme Obesity", NEW ENGLAND JOURNAL OF MEDICINE, Bd. 372, Nr. 1, 1. Januar 2015 (2015-01-01), Seiten 48-54, XP055196767, ISSN: 0028-4793, DOI: 10.1056/NEJMoa1406653 in der Anmeldung erwähnt

• PAMELA FISCHER-POSOVSZKY ET AL: "A New Missense Mutation in the Leptin Gene Causes Mild Obesity and Hypogonadism without Affecting T Cell Responsiveness", JOURNAL OF CLINICAL ENDOCRINOLOGY & METABOLISM, Bd. 95, Nr. 6, 1. Juni 2010 (2010-06-01), Seiten 2836-2840, XP055196771, ISSN: 0021-972X, DOI: 10.1210/jc.2009-2466

• MAZEN I ET AL: "A novel homozygous missense mutation of the leptin gene (N103K) in an obese Egyptian patient", MOLECULAR GENETICS AND METABOLISM, ACADEMIC PRESS, AMSTERDAM, NL, Bd. 97, Nr. 4, 1. August 2009 (2009-08-01) , Seiten 305-308, XP026282979, ISSN: 1096-7192, DOI: 10.1016/J.YMGME.2009.04.002 [gefunden am 2009-04-09]

- JAN-BERND FUNCKE ET AL: "Monogenic forms of childhood obesity due to mutations in the leptin gene", MOLECULAR AND CELLULAR PEDIATRICS, Bd. 168, Nr. 3, 1. Dezember 2014 (2014-12-01), Seiten 1-8, XP055250790, DOI: 10.1186/s40348-014-0003-1

SEQUENCE LISTING

<110> Mediagnost Gesellschaft für Forschung und Herstellung von Diagnostika GmbH

<120> Zusammenstellung von Nachweisreagenzien, In-vitro-Verfahren zum Nachweisen von mutiertem Leptin und Verwendung eines Nachweisreagenzes

<130> E/53284WO/AW/kn

<160> 10

<170> BiSSAP 1.0

<210> 1
<211> 167
<212> PRT
<213> Homo sapiens

<220>
<221> SOURCE
<222> 1..167
<223> /mol_type="protein" /note="Leptin Precursor" /organism="Homo sapiens"

<400> 1

```
Met His Trp Gly Thr Leu Cys Gly Phe Leu Trp Leu Trp Pro Tyr Leu
1               5                   10                  15
Phe Tyr Val Gln Ala Val Pro Ile Gln Lys Val Gln Asp Asp Thr Lys
            20                  25                  30
Thr Leu Ile Lys Thr Ile Val Thr Arg Ile Asn Asp Ile Ser His Thr
            35                  40                  45
Gln Ser Val Ser Ser Lys Gln Lys Val Thr Gly Leu Asp Phe Ile Pro
    50                  55                  60
Gly Leu His Pro Ile Leu Thr Leu Ser Lys Met Asp Gln Thr Leu Ala
65                  70                  75                  80
Val Tyr Gln Gln Ile Leu Thr Ser Met Pro Ser Arg Asn Val Ile Gln
                85                  90                  95
Ile Ser Asn Asp Leu Glu Asn Leu Arg Asp Leu Leu His Val Leu Ala
            100                 105                 110
Phe Ser Lys Ser Cys His Leu Pro Trp Ala Ser Gly Leu Glu Thr Leu
            115                 120                 125
Asp Ser Leu Gly Gly Val Leu Glu Ala Ser Gly Tyr Ser Thr Glu Val
            130                 135                 140
Val Ala Leu Ser Arg Leu Gln Gly Ser Leu Gln Asp Met Leu Trp Gln
145                 150                 155                 160
Leu Asp Leu Ser Pro Gly Cys
                165
```

<210> 2
<211> 167
<212> PRT
<213> Homo sapiens

<220>
<221> SOURCE
<222> 1..167
<223> /mol_type="protein" /note="Leptin Precursor Variante D100Y" /organism="Homo sapiens"

<400> 2

```
Met His Trp Gly Thr Leu Cys Gly Phe Leu Trp Leu Trp Pro Tyr Leu
1               5                   10                  15
Phe Tyr Val Gln Ala Val Pro Ile Gln Lys Val Gln Asp Asp Thr Lys
            20                  25                  30
Thr Leu Ile Lys Thr Ile Val Thr Arg Ile Asn Asp Ile Ser His Thr
            35                  40                  45
Gln Ser Val Ser Ser Lys Gln Lys Val Thr Gly Leu Asp Phe Ile Pro
        50                  55                  60
Gly Leu His Pro Ile Leu Thr Leu Ser Lys Met Asp Gln Thr Leu Ala
65                  70                  75                  80
Val Tyr Gln Gln Ile Leu Thr Ser Met Pro Ser Arg Asn Val Ile Gln
                85                  90                  95
Ile Ser Asn Tyr Leu Glu Asn Leu Arg Asp Leu Leu His Val Leu Ala
            100                 105                 110
Phe Ser Lys Ser Cys His Leu Pro Trp Ala Ser Gly Leu Glu Thr Leu
            115                 120                 125
Asp Ser Leu Gly Gly Val Leu Glu Ala Ser Gly Tyr Ser Thr Glu Val
    130                 135                 140
Val Ala Leu Ser Arg Leu Gln Gly Ser Leu Gln Asp Met Leu Trp Gln
145                 150                 155                 160
Leu Asp Leu Ser Pro Gly Cys
                165
```

<210> 3
<211> 167
<212> PRT
<213> Homo sapiens

<220>
<221> SOURCE
<222> 1..167
<223> /mol_type="protein" /note="Leptin Precursor Variante N103K" /organism="Homo sapiens"

<400> 3

```
Met His Trp Gly Thr Leu Cys Gly Phe Leu Trp Leu Trp Pro Tyr Leu
1               5                   10                  15
Phe Tyr Val Gln Ala Val Pro Ile Gln Lys Val Gln Asp Asp Thr Lys
            20                  25                  30
Thr Leu Ile Lys Thr Ile Val Thr Arg Ile Asn Asp Ile Ser His Thr
            35                  40                  45
Gln Ser Val Ser Ser Lys Gln Lys Val Thr Gly Leu Asp Phe Ile Pro
        50                  55                  60
Gly Leu His Pro Ile Leu Thr Leu Ser Lys Met Asp Gln Thr Leu Ala
65                  70                  75                  80
Val Tyr Gln Gln Ile Leu Thr Ser Met Pro Ser Arg Asn Val Ile Gln
                85                  90                  95
Ile Ser Asn Asp Leu Glu Lys Leu Arg Asp Leu Leu His Val Leu Ala
            100                 105                 110
Phe Ser Lys Ser Cys His Leu Pro Trp Ala Ser Gly Leu Glu Thr Leu
            115                 120                 125
Asp Ser Leu Gly Gly Val Leu Glu Ala Ser Gly Tyr Ser Thr Glu Val
    130                 135                 140
Val Ala Leu Ser Arg Leu Gln Gly Ser Leu Gln Asp Met Leu Trp Gln
145                 150                 155                 160
Leu Asp Leu Ser Pro Gly Cys
                165
```

<210> 4
<211> 167

<210> PRT
<213> Homo sapiens

<220>
<221> SOURCE
<222> 1..167
<223> /mol_type="protein" /note="Leptin Precursor Variante L72S" /organism="Homo sapiens"

<400> 4

```
Met His Trp Gly Thr Leu Cys Gly Phe Leu Trp Leu Trp Pro Tyr Leu
1               5                   10                  15
Phe Tyr Val Gln Ala Val Pro Ile Gln Lys Val Gln Asp Asp Thr Lys
                20                  25                  30
Thr Leu Ile Lys Thr Ile Val Thr Arg Ile Asn Asp Ile Ser His Thr
                35                  40                  45
Gln Ser Val Ser Ser Lys Gln Lys Val Thr Gly Leu Asp Phe Ile Pro
            50                  55                  60
Gly Leu His Pro Ile Leu Thr Ser Ser Lys Met Asp Gln Thr Leu Ala
65                  70                  75                  80
Val Tyr Gln Gln Ile Leu Thr Ser Met Pro Ser Arg Asn Val Ile Gln
                85                  90                  95
Ile Ser Asn Asp Leu Glu Asn Leu Arg Asp Leu Leu His Val Leu Ala
                100                 105                 110
Phe Ser Lys Ser Cys His Leu Pro Trp Ala Ser Gly Leu Glu Thr Leu
            115                 120                 125
Asp Ser Leu Gly Gly Val Leu Glu Ala Ser Gly Tyr Ser Thr Glu Val
            130                 135                 140
Val Ala Leu Ser Arg Leu Gln Gly Ser Leu Gln Asp Met Leu Trp Gln
145                 150                 155                 160
Leu Asp Leu Ser Pro Gly Cys
                165
```

<210> 5
<211> 896
<212> PRT
<213> Homo sapiens

<220>
<221> SOURCE
<222> 1..896
<223> /mol_type="protein" /note="Leptinrezeptor Isoform A" /organism="Homo sapiens"

<400> 5

```
Met Ile Cys Gln Lys Phe Cys Val Val Leu Leu His Trp Glu Phe Ile
1               5                   10                  15
Tyr Val Ile Thr Ala Phe Asn Leu Ser Tyr Pro Ile Thr Pro Trp Arg
            20                  25                  30
Phe Lys Leu Ser Cys Met Pro Pro Asn Ser Thr Tyr Asp Tyr Phe Leu
            35                  40                  45
Leu Pro Ala Gly Leu Ser Lys Asn Thr Ser Asn Ser Asn Gly His Tyr
    50                  55                  60
Glu Thr Ala Val Glu Pro Lys Phe Asn Ser Ser Gly Thr His Phe Ser
65                  70                  75                      80
Asn Leu Ser Lys Thr Thr Phe His Cys Cys Phe Arg Ser Glu Gln Asp
                85                  90                  95
Arg Asn Cys Ser Leu Cys Ala Asp Asn Ile Glu Gly Lys Thr Phe Val
            100                 105                 110
Ser Thr Val Asn Ser Leu Val Phe Gln Gln Ile Asp Ala Asn Trp Asn
        115                 120                 125
Ile Gln Cys Trp Leu Lys Gly Asp Leu Lys Leu Phe Ile Cys Tyr Val
    130                 135                 140
```

```
Glu Ser Leu Phe Lys Asn Leu Phe Arg Asn Tyr Asn Tyr Lys Val His
145                 150                 155                     160
Leu Leu Tyr Val Leu Pro Glu Val Leu Glu Asp Ser Pro Leu Val Pro
                165                 170                 175
Gln Lys Gly Ser Phe Gln Met Val His Cys Asn Cys Ser Val His Glu
            180                 185                 190
Cys Cys Glu Cys Leu Val Pro Val Pro Thr Ala Lys Leu Asn Asp Thr
        195                 200                 205
Leu Leu Met Cys Leu Lys Ile Thr Ser Gly Gly Val Ile Phe Gln Ser
        210                 215                 220
Pro Leu Met Ser Val Gln Pro Ile Asn Met Val Lys Pro Asp Pro Pro
225                 230                 235                     240
Leu Gly Leu His Met Glu Ile Thr Asp Asp Gly Asn Leu Lys Ile Ser
                245                 250                 255
Trp Ser Ser Pro Pro Leu Val Pro Phe Pro Leu Gln Tyr Gln Val Lys
            260                 265                 270
Tyr Ser Glu Asn Ser Thr Thr Val Ile Arg Glu Ala Asp Lys Ile Val
            275                 280                 285
Ser Ala Thr Ser Leu Leu Val Asp Ser Ile Leu Pro Gly Ser Ser Tyr
        290                 295                 300
Glu Val Gln Val Arg Gly Lys Arg Leu Asp Gly Pro Gly Ile Trp Ser
305                 310                 315                     320
Asp Trp Ser Thr Pro Arg Val Phe Thr Thr Gln Asp Val Ile Tyr Phe
                325                 330                 335
Pro Pro Lys Ile Leu Thr Ser Val Gly Ser Asn Val Ser Phe His Cys
            340                 345                 350
Ile Tyr Lys Lys Glu Asn Lys Ile Val Pro Ser Lys Glu Ile Val Trp
            355                 360                 365
Trp Met Asn Leu Ala Glu Lys Ile Pro Gln Ser Gln Tyr Asp Val Val
        370                 375                 380
Ser Asp His Val Ser Lys Val Thr Phe Phe Asn Leu Asn Glu Thr Lys
385                 390                 395                     400
Pro Arg Gly Lys Phe Thr Tyr Asp Ala Val Tyr Cys Cys Asn Glu His
                405                 410                 415
Glu Cys His His Arg Tyr Ala Glu Leu Tyr Val Ile Asp Val Asn Ile
            420                 425                 430
Asn Ile Ser Cys Glu Thr Asp Gly Tyr Leu Thr Lys Met Thr Cys Arg
            435                 440                 445
Trp Ser Thr Ser Thr Ile Gln Ser Leu Ala Glu Ser Thr Leu Gln Leu
        450                 455                 460
Arg Tyr His Arg Ser Ser Leu Tyr Cys Ser Asp Ile Pro Ser Ile His
465                 470                 475                     480
Pro Ile Ser Glu Pro Lys Asp Cys Tyr Leu Gln Ser Asp Gly Phe Tyr
                485                 490                 495
Glu Cys Ile Phe Gln Pro Ile Phe Leu Leu Ser Gly Tyr Thr Met Trp
            500                 505                 510
Ile Arg Ile Asn His Ser Leu Gly Ser Leu Asp Ser Pro Pro Thr Cys
            515                 520                 525
Val Leu Pro Asp Ser Val Val Lys Pro Leu Pro Pro Ser Ser Val Lys
        530                 535                 540
Ala Glu Ile Thr Ile Asn Ile Gly Leu Leu Lys Ile Ser Trp Glu Lys
545                 550                 555                     560
Pro Val Phe Pro Glu Asn Asn Leu Gln Phe Gln Ile Arg Tyr Gly Leu
                565                 570                 575
Ser Gly Lys Glu Val Gln Trp Lys Met Tyr Glu Val Tyr Asp Ala Lys
            580                 585                 590
Ser Lys Ser Val Ser Leu Pro Val Pro Asp Leu Cys Ala Val Tyr Ala
            595                 600                 605
Val Gln Val Arg Cys Lys Arg Leu Asp Gly Leu Gly Tyr Trp Ser Asn
        610                 615                 620
Trp Ser Asn Pro Ala Tyr Thr Val Val Met Asp Ile Lys Val Pro Met
625                 630                 635                     640
Arg Gly Pro Glu Phe Trp Arg Ile Ile Asn Gly Asp Thr Met Lys Lys
```

```
                              645                     650                     655
         Glu Lys Asn Val Thr Leu Leu Trp Lys Pro Leu Met Lys Asn Asp Ser
                      660                     665                     670
         Leu Cys Ser Val Gln Arg Tyr Val Ile Asn His His Thr Ser Cys Asn
                      675                     680                     685
         Gly Thr Trp Ser Glu Asp Val Gly Asn His Thr Lys Phe Thr Phe Leu
             690                     695                     700
         Trp Thr Glu Gln Ala His Thr Val Thr Val Leu Ala Ile Asn Ser Ile
         705                     710                     715                     720
         Gly Ala Ser Val Ala Asn Phe Asn Leu Thr Phe Ser Trp Pro Met Ser
                      725                     730                     735
         Lys Val Asn Ile Val Gln Ser Leu Ser Ala Tyr Pro Leu Asn Ser Ser
                      740                     745                     750
         Cys Val Ile Val Ser Trp Ile Leu Ser Pro Ser Asp Tyr Lys Leu Met
                      755                     760                     765
         Tyr Phe Ile Ile Glu Trp Lys Asn Leu Asn Glu Asp Gly Glu Ile Lys
             770                     775                     780
         Trp Leu Arg Ile Ser Ser Ser Val Lys Lys Tyr Tyr Ile His Asp His
         785                     790                     795                     800
         Phe Ile Pro Ile Glu Lys Tyr Gln Phe Ser Leu Tyr Pro Ile Phe Met
                      805                     810                     815
         Glu Gly Val Gly Lys Pro Lys Ile Ile Asn Ser Phe Thr Gln Asp Asp
                      820                     825                     830
         Ile Glu Lys His Gln Ser Asp Ala Gly Leu Tyr Val Ile Val Pro Val
                      835                     840                     845
         Ile Ile Ser Ser Ser Ile Leu Leu Leu Gly Thr Leu Leu Ile Ser His
                      850                     855                     860
         Gln Arg Met Lys Lys Leu Phe Trp Glu Asp Val Pro Asn Pro Lys Asn
         865                     870                     875                     880
         Cys Ser Trp Ala Gln Gly Leu Asn Phe Gln Lys Arg Thr Asp Ile Leu
                      885                     890                     895
```

<210> 6
<211> 1165
<212> PRT
<213> Homo sapiens

<220>
<221> SOURCE
<222> 1..1165
<223> /mol_type="protein" /note="Leptinrezeptor Isoform B" /organism="Homo sapiens"

<400> 6

```
Met Ile Cys Gln Lys Phe Cys Val Val Leu Leu His Trp Glu Phe Ile
1               5                   10                  15
Tyr Val Ile Thr Ala Phe Asn Leu Ser Tyr Pro Ile Thr Pro Trp Arg
            20                  25                  30
Phe Lys Leu Ser Cys Met Pro Pro Asn Ser Thr Tyr Asp Tyr Phe Leu
            35                  40                  45
Leu Pro Ala Gly Leu Ser Lys Asn Thr Ser Asn Ser Asn Gly His Tyr
    50                  55                  60
Glu Thr Ala Val Glu Pro Lys Phe Asn Ser Ser Gly Thr His Phe Ser
65                  70                  75                  80
Asn Leu Ser Lys Thr Thr Phe His Cys Cys Phe Arg Ser Glu Gln Asp
            85                  90                  95
Arg Asn Cys Ser Leu Cys Ala Asp Asn Ile Glu Gly Lys Thr Phe Val
            100                 105                 110
Ser Thr Val Asn Ser Leu Val Phe Gln Gln Ile Asp Ala Asn Trp Asn
            115                 120                 125
Ile Gln Cys Trp Leu Lys Gly Asp Leu Lys Leu Phe Ile Cys Tyr Val
    130                 135                 140
```

Glu Ser Leu Phe Lys Asn Leu Phe Arg Asn Tyr Asn Tyr Lys Val His
145                     150                 155                     160
Leu Leu Tyr Val Leu Pro Glu Val Leu Glu Asp Ser Pro Leu Val Pro
                165                 170                     175
Gln Lys Gly Ser Phe Gln Met Val His Cys Asn Cys Ser Val His Glu
                180                 185                     190
Cys Cys Glu Cys Leu Val Pro Val Pro Thr Ala Lys Leu Asn Asp Thr
            195                 200                 205
Leu Leu Met Cys Leu Lys Ile Thr Ser Gly Gly Val Ile Phe Gln Ser
        210                 215                 220
Pro Leu Met Ser Val Gln Pro Ile Asn Met Val Lys Pro Asp Pro Pro
225                 230                 235                     240
Leu Gly Leu His Met Glu Ile Thr Asp Asp Gly Asn Leu Lys Ile Ser
                245                 250                     255
Trp Ser Ser Pro Pro Leu Val Pro Phe Pro Leu Gln Tyr Gln Val Lys
            260                 265                 270
Tyr Ser Glu Asn Ser Thr Thr Val Ile Arg Glu Ala Asp Lys Ile Val
        275                 280                 285
Ser Ala Thr Ser Leu Leu Val Asp Ser Ile Leu Pro Gly Ser Ser Tyr
        290                 295                 300
Glu Val Gln Val Arg Gly Lys Arg Leu Asp Gly Pro Gly Ile Trp Ser
305                 310                 315                     320
Asp Trp Ser Thr Pro Arg Val Phe Thr Thr Gln Asp Val Ile Tyr Phe
            325                 330                     335
Pro Pro Lys Ile Leu Thr Ser Val Gly Ser Asn Val Ser Phe His Cys
            340                 345                 350
Ile Tyr Lys Lys Glu Asn Lys Ile Val Pro Ser Lys Glu Ile Val Trp
        355                 360                 365
Trp Met Asn Leu Ala Glu Lys Ile Pro Gln Ser Gln Tyr Asp Val Val
        370                 375                 380
Ser Asp His Val Ser Lys Val Thr Phe Phe Asn Leu Asn Glu Thr Lys
385                 390                 395                     400
Pro Arg Gly Lys Phe Thr Tyr Asp Ala Val Tyr Cys Cys Asn Glu His
                405                 410                     415
Glu Cys His His Arg Tyr Ala Glu Leu Tyr Val Ile Asp Val Asn Ile
            420                 425                 430
Asn Ile Ser Cys Glu Thr Asp Gly Tyr Leu Thr Lys Met Thr Cys Arg
            435                 440                 445
Trp Ser Thr Ser Thr Ile Gln Ser Leu Ala Glu Ser Thr Leu Gln Leu
        450                 455                 460
Arg Tyr His Arg Ser Ser Leu Tyr Cys Ser Asp Ile Pro Ser Ile His
465                 470                 475                     480
Pro Ile Ser Glu Pro Lys Asp Cys Tyr Leu Gln Ser Asp Gly Phe Tyr
            485                 490                     495
Glu Cys Ile Phe Gln Pro Ile Phe Leu Leu Ser Gly Tyr Thr Met Trp
            500                 505                 510
Ile Arg Ile Asn His Ser Leu Gly Ser Leu Asp Ser Pro Pro Thr Cys
        515                 520                 525
Val Leu Pro Asp Ser Val Val Lys Pro Leu Pro Pro Ser Ser Val Lys
        530                 535                 540
Ala Glu Ile Thr Ile Asn Ile Gly Leu Leu Lys Ile Ser Trp Glu Lys
545                 550                 555                     560
Pro Val Phe Pro Glu Asn Asn Leu Gln Phe Gln Ile Arg Tyr Gly Leu
                565                 570                     575
Ser Gly Lys Glu Val Gln Trp Lys Met Tyr Glu Val Tyr Asp Ala Lys
                580                 585                     590
Ser Lys Ser Val Ser Leu Pro Val Pro Asp Leu Cys Ala Val Tyr Ala
            595                 600                 605
Val Gln Val Arg Cys Lys Arg Leu Asp Gly Leu Gly Tyr Trp Ser Asn
610                 615                 620
Trp Ser Asn Pro Ala Tyr Thr Val Val Met Asp Ile Lys Val Pro Met
625                 630                 635                     640
Arg Gly Pro Glu Phe Trp Arg Ile Ile Asn Gly Asp Thr Met Lys Lys

23

```
                         645                    650                    655
         Glu Lys Asn Val Thr Leu Leu Trp Lys Pro Leu Met Lys Asn Asp Ser
                     660                    665                    670
         Leu Cys Ser Val Gln Arg Tyr Val Ile Asn His His Thr Ser Cys Asn
                     675                    680                    685
         Gly Thr Trp Ser Glu Asp Val Gly Asn His Thr Lys Phe Thr Phe Leu
                     690                    695                    700
         Trp Thr Glu Gln Ala His Thr Val Thr Val Leu Ala Ile Asn Ser Ile
         705                    710                    715                    720
         Gly Ala Ser Val Ala Asn Phe Asn Leu Thr Phe Ser Trp Pro Met Ser
                     725                    730                    735
         Lys Val Asn Ile Val Gln Ser Leu Ser Ala Tyr Pro Leu Asn Ser Ser
                     740                    745                    750
         Cys Val Ile Val Ser Trp Ile Leu Ser Pro Ser Asp Tyr Lys Leu Met
                     755                    760                    765
         Tyr Phe Ile Ile Glu Trp Lys Asn Leu Asn Glu Asp Gly Glu Ile Lys
                     770                    775                    780
         Trp Leu Arg Ile Ser Ser Ser Val Lys Lys Tyr Tyr Ile His Asp His
         785                    790                    795                    800
         Phe Ile Pro Ile Glu Lys Tyr Gln Phe Ser Leu Tyr Pro Ile Phe Met
                     805                    810                    815
         Glu Gly Val Gly Lys Pro Lys Ile Ile Asn Ser Phe Thr Gln Asp Asp
                     820                    825                    830
         Ile Glu Lys His Gln Ser Asp Ala Gly Leu Tyr Val Ile Val Pro Val
                     835                    840                    845
         Ile Ile Ser Ser Ser Ile Leu Leu Leu Gly Thr Leu Leu Ile Ser His
                     850                    855                    860
         Gln Arg Met Lys Lys Leu Phe Trp Glu Asp Val Pro Asn Pro Lys Asn
         865                    870                    875                    880
         Cys Ser Trp Ala Gln Gly Leu Asn Phe Gln Lys Pro Glu Thr Phe Glu
                     885                    890                    895
         His Leu Phe Ile Lys His Thr Ala Ser Val Thr Cys Gly Pro Leu Leu
                     900                    905                    910
         Leu Glu Pro Glu Thr Ile Ser Glu Asp Ile Ser Val Asp Thr Ser Trp
                     915                    920                    925
         Lys Asn Lys Asp Glu Met Met Pro Thr Thr Val Val Ser Leu Leu Ser
                     930                    935                    940
         Thr Thr Asp Leu Glu Lys Gly Ser Val Cys Ile Ser Asp Gln Phe Asn
         945                    950                    955                    960
         Ser Val Asn Phe Ser Glu Ala Glu Gly Thr Glu Val Thr Tyr Glu Asp
                     965                    970                    975
         Glu Ser Gln Arg Gln Pro Phe Val Lys Tyr Ala Thr Leu Ile Ser Asn
                     980                    985                    990
         Ser Lys Pro Ser Glu Thr Gly Glu Glu Gln Gly Leu Ile Asn Ser Ser
                     995                    1000                   1005
         Val Thr Lys Cys Phe Ser Ser Lys Asn Ser Pro Leu Lys Asp Ser Phe
                     1010                   1015                   1020
         Ser Asn Ser Ser Trp Glu Ile Glu Ala Gln Ala Phe Phe Ile Leu Ser
         1025                   1030                   1035                   1040
         Asp Gln His Pro Asn Ile Ile Ser Pro His Leu Thr Phe Ser Glu Gly
                     1045                   1050                   1055
         Leu Asp Glu Leu Leu Lys Leu Glu Gly Asn Phe Pro Glu Glu Asn Asn
                     1060                   1065                   1070
         Asp Lys Lys Ser Ile Tyr Tyr Leu Gly Val Thr Ser Ile Lys Lys Arg
                     1075                   1080                   1085
         Glu Ser Gly Val Leu Leu Thr Asp Lys Ser Arg Val Ser Cys Pro Phe
                     1090                   1095                   1100
         Pro Ala Pro Cys Leu Phe Thr Asp Ile Arg Val Leu Gln Asp Ser Cys
         1105                   1110                   1115                   1120
         Ser His Phe Val Glu Asn Asn Ile Asn Leu Gly Thr Ser Ser Lys Lys
                     1125                   1130                   1135
         Thr Phe Ala Ser Tyr Met Pro Gln Phe Gln Thr Cys Ser Thr Gln Thr
                     1140                   1145                   1150
```

24

```
His Lys Ile Met Glu Asn Lys Met Cys Asp Leu Thr Val
        1155                1160                1165
```

<210> 7
<211> 958
<212> PRT
<213> Homo sapiens

<220>
<221> SOURCE
<222> 1..958
<223> /mol_type="protein" /note="Leptinrezeptor Isoform C" /organism="Homo sapiens"

<400> 7

```
His Lys Ile Met Glu Asn Lys Met Cys Asp Leu Thr Val
        1155                1160                1165
```

```
Met Ile Cys Gln Lys Phe Cys Val Val Leu Leu His Trp Glu Phe Ile
1               5                   10                  15
Tyr Val Ile Thr Ala Phe Asn Leu Ser Tyr Pro Ile Thr Pro Trp Arg
            20              25                  30
Phe Lys Leu Ser Cys Met Pro Pro Asn Ser Thr Tyr Asp Tyr Phe Leu
            35              40                  45
Leu Pro Ala Gly Leu Ser Lys Asn Thr Ser Asn Ser Asn Gly His Tyr
        50              55                  60
Glu Thr Ala Val Glu Pro Lys Phe Asn Ser Ser Gly Thr His Phe Ser
65                  70                  75                  80
Asn Leu Ser Lys Thr Thr Phe His Cys Cys Phe Arg Ser Glu Gln Asp
                85                  90                  95
Arg Asn Cys Ser Leu Cys Ala Asp Asn Ile Glu Gly Lys Thr Phe Val
            100                 105                 110
Ser Thr Val Asn Ser Leu Val Phe Gln Gln Ile Asp Ala Asn Trp Asn
        115                 120                 125
Ile Gln Cys Trp Leu Lys Gly Asp Leu Lys Leu Phe Ile Cys Tyr Val
    130                 135                 140
Glu Ser Leu Phe Lys Asn Leu Phe Arg Asn Tyr Asn Tyr Lys Val His
145                 150                 155                 160
Leu Leu Tyr Val Leu Pro Glu Val Leu Glu Asp Ser Pro Leu Val Pro
                165                 170                 175
Gln Lys Gly Ser Phe Gln Met Val His Cys Asn Cys Ser Val His Glu
            180                 185                 190
Cys Cys Glu Cys Leu Val Pro Val Pro Thr Ala Lys Leu Asn Asp Thr
            195                 200                 205
Leu Leu Met Cys Leu Lys Ile Thr Ser Gly Gly Val Ile Phe Gln Ser
    210                 215                 220
Pro Leu Met Ser Val Gln Pro Ile Asn Met Val Lys Pro Asp Pro Pro
225                 230                 235                 240
Leu Gly Leu His Met Glu Ile Thr Asp Asp Gly Asn Leu Lys Ile Ser
                245                 250                 255
Trp Ser Ser Pro Pro Leu Val Pro Phe Pro Leu Gln Tyr Gln Val Lys
            260                 265                 270
Tyr Ser Glu Asn Ser Thr Thr Val Ile Arg Glu Ala Asp Lys Ile Val
            275                 280                 285
Ser Ala Thr Ser Leu Leu Val Asp Ser Ile Leu Pro Gly Ser Ser Tyr
    290                 295                 300
Glu Val Gln Val Arg Gly Lys Arg Leu Asp Gly Pro Gly Ile Trp Ser
305                 310                 315                 320
Asp Trp Ser Thr Pro Arg Val Phe Thr Thr Gln Asp Val Ile Tyr Phe
            325                 330                 335
Pro Pro Lys Ile Leu Thr Ser Val Gly Ser Asn Val Ser Phe His Cys
            340                 345                 350
Ile Tyr Lys Lys Glu Asn Lys Ile Val Pro Ser Lys Glu Ile Val Trp
    355                 360                 365
Trp Met Asn Leu Ala Glu Lys Ile Pro Gln Ser Gln Tyr Asp Val Val
```

```
                370                           375                           380
      Ser Asp His Val Ser Lys Val Thr Phe Phe Asn Leu Asn Glu Thr Lys
      385                           390                           395                           400
      Pro Arg Gly Lys Phe Thr Tyr Asp Ala Val Tyr Cys Cys Asn Glu His
                                405                           410                           415
      Glu Cys His His Arg Tyr Ala Glu Leu Tyr Val Ile Asp Val Asn Ile
                          420                           425                           430
      Asn Ile Ser Cys Glu Thr Asp Gly Tyr Leu Thr Lys Met Thr Cys Arg
                    435                           440                           445
      Trp Ser Thr Ser Thr Ile Gln Ser Leu Ala Glu Ser Thr Leu Gln Leu
                450                           455                           460
      Arg Tyr His Arg Ser Ser Leu Tyr Cys Ser Asp Ile Pro Ser Ile His
      465                           470                           475                           480
      Pro Ile Ser Glu Pro Lys Asp Cys Tyr Leu Gln Ser Asp Gly Phe Tyr
                                485                           490                           495
      Glu Cys Ile Phe Gln Pro Ile Phe Leu Leu Ser Gly Tyr Thr Met Trp
                          500                           505                           510
      Ile Arg Ile Asn His Ser Leu Gly Ser Leu Asp Ser Pro Thr Cys
                    515                           520                           525
      Val Leu Pro Asp Ser Val Val Lys Pro Leu Pro Pro Ser Ser Val Lys
                    530                           535                           540
      Ala Glu Ile Thr Ile Asn Ile Gly Leu Leu Lys Ile Ser Trp Glu Lys
      545                           550                           555                           560
      Pro Val Phe Pro Glu Asn Asn Leu Gln Phe Gln Ile Arg Tyr Gly Leu
                                565                           570                           575
      Ser Gly Lys Glu Val Gln Trp Lys Met Tyr Glu Val Tyr Asp Ala Lys
                          580                           585                           590
      Ser Lys Ser Val Ser Leu Pro Val Pro Asp Leu Cys Ala Val Tyr Ala
                    595                           600                           605
      Val Gln Val Arg Cys Lys Arg Leu Asp Gly Leu Gly Tyr Trp Ser Asn
                    610                           615                           620
      Trp Ser Asn Pro Ala Tyr Thr Val Val Met Asp Ile Lys Val Pro Met
      625                           630                           635                           640
      Arg Gly Pro Glu Phe Trp Arg Ile Ile Asn Gly Asp Thr Met Lys Lys
                                645                           650                           655
      Glu Lys Asn Val Thr Leu Leu Trp Lys Pro Leu Met Lys Asn Asp Ser
                          660                           665                           670
      Leu Cys Ser Val Gln Arg Tyr Val Ile Asn His His Thr Ser Cys Asn
                    675                           680                           685
      Gly Thr Trp Ser Glu Asp Val Gly Asn His Thr Lys Phe Thr Phe Leu
                690                           695                           700
      Trp Thr Glu Gln Ala His Thr Val Thr Val Leu Ala Ile Asn Ser Ile
      705                           710                           715                           720
      Gly Ala Ser Val Ala Asn Phe Asn Leu Thr Phe Ser Trp Pro Met Ser
                                725                           730                           735
      Lys Val Asn Ile Val Gln Ser Leu Ser Ala Tyr Pro Leu Asn Ser Ser
                          740                           745                           750
      Cys Val Ile Val Ser Trp Ile Leu Ser Pro Ser Asp Tyr Lys Leu Met
                    755                           760                           765
      Tyr Phe Ile Ile Glu Trp Lys Asn Leu Asn Glu Asp Gly Glu Ile Lys
                770                           775                           780
      Trp Leu Arg Ile Ser Ser Ser Val Lys Lys Tyr Tyr Ile His Asp His
      785                           790                           795                           800
      Phe Ile Pro Ile Glu Lys Tyr Gln Phe Ser Leu Tyr Pro Ile Phe Met
                                805                           810                           815
      Glu Gly Val Gly Lys Pro Lys Ile Ile Asn Ser Phe Thr Gln Asp Asp
                          820                           825                           830
      Ile Glu Lys His Gln Ser Asp Ala Gly Leu Tyr Val Ile Val Pro Val
                    835                           840                           845
      Ile Ile Ser Ser Ser Ile Leu Leu Leu Gly Thr Leu Leu Ile Ser His
                    850                           855                           860
      Gln Arg Met Lys Lys Leu Phe Trp Glu Asp Val Pro Asn Pro Lys Asn
      865                           870                           875                           880
```

```
Cys Ser Trp Ala Gln Gly Leu Asn Phe Gln Lys Met Leu Glu Gly Ser
                885                     890                     895
Met Phe Val Lys Ser His His His Ser Leu Ile Ser Ser Thr Gln Gly
                900                     905                     910
His Lys His Cys Gly Arg Pro Gln Gly Pro Leu His Arg Lys Thr Arg
                915                     920                     925
Asp Leu Cys Ser Leu Val Tyr Leu Leu Thr Leu Pro Pro Leu Leu Ser
                930                     935                     940
Tyr Asp Pro Ala Lys Ser Pro Ser Val Arg Asn Thr Gln Glu
945                     950                     955
```

<210> 8
<211> 906
<212> PRT
<213> Homo sapiens

<220>
<221> SOURCE
<222> 1..906
<223> /mol_type="protein" /note="Leptinrezeptor Isoform D" /organism="Homo sapiens"

<400> 8

```
Met Ile Cys Gln Lys Phe Cys Val Val Leu Leu His Trp Glu Phe Ile
1                   5                   10                  15
Tyr Val Ile Thr Ala Phe Asn Leu Ser Tyr Pro Ile Thr Pro Trp Arg
            20                  25                  30
Phe Lys Leu Ser Cys Met Pro Pro Asn Ser Thr Tyr Asp Tyr Phe Leu
            35                  40                  45
Leu Pro Ala Gly Leu Ser Lys Asn Thr Ser Asn Ser Asn Gly His Tyr
    50                  55                  60
Glu Thr Ala Val Glu Pro Lys Phe Asn Ser Ser Gly Thr His Phe Ser
65                  70                  75                  80
Asn Leu Ser Lys Thr Thr Phe His Cys Cys Phe Arg Ser Glu Gln Asp
            85                  90                  95
Arg Asn Cys Ser Leu Cys Ala Asp Asn Ile Glu Gly Lys Thr Phe Val
            100                 105                 110
Ser Thr Val Asn Ser Leu Val Phe Gln Gln Ile Asp Ala Asn Trp Asn
            115                 120                 125
Ile Gln Cys Trp Leu Lys Gly Asp Leu Lys Leu Phe Ile Cys Tyr Val
    130                 135                 140
Glu Ser Leu Phe Lys Asn Leu Phe Arg Asn Tyr Asn Tyr Lys Val His
145                 150                 155                 160
Leu Leu Tyr Val Leu Pro Glu Val Leu Glu Asp Ser Pro Leu Val Pro
            165                 170                 175
Gln Lys Gly Ser Phe Gln Met Val His Cys Asn Cys Ser Val His Glu
            180                 185                 190
Cys Cys Glu Cys Leu Val Pro Val Pro Thr Ala Lys Leu Asn Asp Thr
            195                 200                 205
Leu Leu Met Cys Leu Lys Ile Thr Ser Gly Gly Val Ile Phe Gln Ser
    210                 215                 220
Pro Leu Met Ser Val Gln Pro Ile Asn Met Val Lys Pro Asp Pro Pro
225                 230                 235                 240
Leu Gly Leu His Met Glu Ile Thr Asp Asp Gly Asn Leu Lys Ile Ser
            245                 250                 255
Trp Ser Ser Pro Pro Leu Val Pro Phe Pro Leu Gln Tyr Gln Val Lys
            260                 265                 270
Tyr Ser Glu Asn Ser Thr Thr Val Ile Arg Glu Ala Asp Lys Ile Val
            275                 280                 285
Ser Ala Thr Ser Leu Leu Val Asp Ser Ile Leu Pro Gly Ser Ser Tyr
    290                 295                 300
Glu Val Gln Val Arg Gly Lys Arg Leu Asp Gly Pro Gly Ile Trp Ser
```

29

```
            305                         310                         315                         320
            Asp Trp Ser Thr Pro Arg Val Phe Thr Thr Gln Asp Val Ile Tyr Phe
                            325                         330                         335
            Pro Pro Lys Ile Leu Thr Ser Val Gly Ser Asn Val Ser Phe His Cys
                            340                         345                         350
            Ile Tyr Lys Lys Glu Asn Lys Ile Val Pro Ser Lys Glu Ile Val Trp
                            355                         360                         365
            Trp Met Asn Leu Ala Glu Lys Ile Pro Gln Ser Gln Tyr Asp Val Val
                        370                         375                         380
            Ser Asp His Val Ser Lys Val Thr Phe Phe Asn Leu Asn Glu Thr Lys
            385                         390                         395                         400
            Pro Arg Gly Lys Phe Thr Tyr Asp Ala Val Tyr Cys Cys Asn Glu His
                            405                         410                         415
            Glu Cys His His Arg Tyr Ala Glu Leu Tyr Val Ile Asp Val Asn Ile
                            420                         425                         430
            Asn Ile Ser Cys Glu Thr Asp Gly Tyr Leu Thr Lys Met Thr Cys Arg
                            435                         440                         445
            Trp Ser Thr Ser Thr Ile Gln Ser Leu Ala Glu Ser Thr Leu Gln Leu
                        450                         455                         460
            Arg Tyr His Arg Ser Ser Leu Tyr Cys Ser Asp Ile Pro Ser Ile His
            465                         470                         475                         480
            Pro Ile Ser Glu Pro Lys Asp Cys Tyr Leu Gln Ser Asp Gly Phe Tyr
                            485                         490                         495
            Glu Cys Ile Phe Gln Pro Ile Phe Leu Leu Ser Gly Tyr Thr Met Trp
                        500                         505                         510
            Ile Arg Ile Asn His Ser Leu Gly Ser Leu Asp Ser Pro Thr Cys
                        515                         520                         525
            Val Leu Pro Asp Ser Val Val Lys Pro Leu Pro Pro Ser Ser Val Lys
                        530                         535                         540
            Ala Glu Ile Thr Ile Asn Ile Gly Leu Leu Lys Ile Ser Trp Glu Lys
            545                         550                         555                         560
            Pro Val Phe Pro Glu Asn Asn Leu Gln Phe Gln Ile Arg Tyr Gly Leu
                            565                         570                         575
            Ser Gly Lys Glu Val Gln Trp Lys Met Tyr Glu Val Tyr Asp Ala Lys
                        580                         585                         590
            Ser Lys Ser Val Ser Leu Pro Val Pro Asp Leu Cys Ala Val Tyr Ala
                        595                         600                         605
            Val Gln Val Arg Cys Lys Arg Leu Asp Gly Leu Gly Tyr Trp Ser Asn
                        610                         615                         620
            Trp Ser Asn Pro Ala Tyr Thr Val Val Met Asp Ile Lys Val Pro Met
            625                         630                         635                         640
            Arg Gly Pro Glu Phe Trp Arg Ile Ile Asn Gly Asp Thr Met Lys Lys
                            645                         650                         655
            Glu Lys Asn Val Thr Leu Leu Trp Lys Pro Leu Met Lys Asn Asp Ser
                        660                         665                         670
            Leu Cys Ser Val Gln Arg Tyr Val Ile Asn His His Thr Ser Cys Asn
                        675                         680                         685
            Gly Thr Trp Ser Glu Asp Val Gly Asn His Thr Lys Phe Thr Phe Leu
                        690                         695                         700
            Trp Thr Glu Gln Ala His Thr Val Thr Val Leu Ala Ile Asn Ser Ile
            705                         710                         715                         720
            Gly Ala Ser Val Ala Asn Phe Asn Leu Thr Phe Ser Trp Pro Met Ser
                            725                         730                         735
            Lys Val Asn Ile Val Gln Ser Leu Ser Ala Tyr Pro Leu Asn Ser Ser
                        740                         745                         750
            Cys Val Ile Val Ser Trp Ile Leu Ser Pro Ser Asp Tyr Lys Leu Met
                        755                         760                         765
            Tyr Phe Ile Ile Glu Trp Lys Asn Leu Asn Glu Asp Gly Glu Ile Lys
                        770                         775                         780
            Trp Leu Arg Ile Ser Ser Ser Val Lys Lys Tyr Tyr Ile His Asp His
            785                         790                         795                         800
            Phe Ile Pro Ile Glu Lys Tyr Gln Phe Ser Leu Tyr Pro Ile Phe Met
                            805                         810                         815
```

30

```
        Glu Gly Val Gly Lys Pro Lys Ile Ile Asn Ser Phe Thr Gln Asp Asp
                    820                 825                 830
        Ile Glu Lys His Gln Ser Asp Ala Gly Leu Tyr Val Ile Val Pro Val
                    835                 840                 845
        Ile Ile Ser Ser Ser Ile Leu Leu Leu Gly Thr Leu Leu Ile Ser His
            850                 855                 860
        Gln Arg Met Lys Lys Leu Phe Trp Glu Asp Val Pro Asn Pro Lys Asn
        865                 870                 875                 880
        Cys Ser Trp Ala Gln Gly Leu Asn Phe Gln Lys Lys Met Pro Gly Thr
                    885                 890                 895
        Lys Glu Leu Leu Gly Gly Gly Trp Leu Thr
                    900                 905
```

<210> 9
<211> 839
<212> PRT
<213> Homo sapiens

<220>
<221> SOURCE
<222> 1..839
<223> /mol_type="protein" /note="Leptinrezeptor Isoform E" /organism="Homo sapiens"

<400> 9

```
Met Ile Cys Gln Lys Phe Cys Val Val Leu Leu His Trp Glu Phe Ile
1               5                   10                  15
Tyr Val Ile Thr Ala Phe Asn Leu Ser Tyr Pro Ile Thr Pro Trp Arg
            20                  25                  30
Phe Lys Leu Ser Cys Met Pro Pro Asn Ser Thr Tyr Asp Tyr Phe Leu
            35                  40                  45
Leu Pro Ala Gly Leu Ser Lys Asn Thr Ser Asn Ser Asn Gly His Tyr
    50                  55                  60
Glu Thr Ala Val Glu Pro Lys Phe Asn Ser Ser Gly Thr His Phe Ser
65                  70                  75                  80
Asn Leu Ser Lys Thr Thr Phe His Cys Cys Phe Arg Ser Glu Gln Asp
            85                  90                  95
Arg Asn Cys Ser Leu Cys Ala Asp Asn Ile Glu Gly Lys Thr Phe Val
        100                 105                 110
Ser Thr Val Asn Ser Leu Val Phe Gln Gln Ile Asp Ala Asn Trp Asn
        115                 120                 125
Ile Gln Cys Trp Leu Lys Gly Asp Leu Lys Leu Phe Ile Cys Tyr Val
    130                 135                 140
Glu Ser Leu Phe Lys Asn Leu Phe Arg Asn Tyr Asn Tyr Lys Val His
145                 150                 155                 160
Leu Leu Tyr Val Leu Pro Glu Val Leu Glu Asp Ser Pro Leu Val Pro
            165                 170                 175
Gln Lys Gly Ser Phe Gln Met Val His Cys Asn Cys Ser Val His Glu
            180                 185                 190
Cys Cys Glu Cys Leu Val Pro Val Pro Thr Ala Lys Leu Asn Asp Thr
        195                 200                 205
Leu Leu Met Cys Leu Lys Ile Thr Ser Gly Gly Val Ile Phe Gln Ser
    210                 215                 220
Pro Leu Met Ser Val Gln Pro Ile Asn Met Val Lys Pro Asp Pro Pro
225                 230                 235                 240
Leu Gly Leu His Met Glu Ile Thr Asp Asp Gly Asn Leu Lys Ile Ser
            245                 250                 255
Trp Ser Ser Pro Pro Leu Val Pro Phe Pro Leu Gln Tyr Gln Val Lys
            260                 265                 270
Tyr Ser Glu Asn Ser Thr Thr Val Ile Arg Glu Ala Asp Lys Ile Val
    275                 280                 285
Ser Ala Thr Ser Leu Leu Val Asp Ser Ile Leu Pro Gly Ser Ser Tyr
```

32

```
            290                    295                    300
Glu Val Gln Val Arg Gly Lys Arg Leu Asp Gly Pro Gly Ile Trp Ser
305                    310                    315                    320
Asp Trp Ser Thr Pro Arg Val Phe Thr Thr Gln Asp Val Ile Tyr Phe
                325                    330                    335
Pro Pro Lys Ile Leu Thr Ser Val Gly Ser Asn Val Ser Phe His Cys
            340                    345                    350
Ile Tyr Lys Lys Glu Asn Lys Ile Val Pro Ser Lys Glu Ile Val Trp
            355                    360                    365
Trp Met Asn Leu Ala Glu Lys Ile Pro Gln Ser Gln Tyr Asp Val Val
            370                    375                    380
Ser Asp His Val Ser Lys Val Thr Phe Phe Asn Leu Asn Glu Thr Lys
385                    390                    395                    400
Pro Arg Gly Lys Phe Thr Tyr Asp Ala Val Tyr Cys Cys Asn Glu His
                405                    410                    415
Glu Cys His His Arg Tyr Ala Glu Leu Tyr Val Ile Asp Val Asn Ile
            420                    425                    430
Asn Ile Ser Cys Glu Thr Asp Gly Tyr Leu Thr Lys Met Thr Cys Arg
            435                    440                    445
Trp Ser Thr Ser Thr Ile Gln Ser Leu Ala Glu Ser Thr Leu Gln Leu
            450                    455                    460
Arg Tyr His Arg Ser Ser Leu Tyr Cys Ser Asp Ile Pro Ser Ile His
465                    470                    475                    480
Pro Ile Ser Glu Pro Lys Asp Cys Tyr Leu Gln Ser Asp Gly Phe Tyr
                485                    490                    495
Glu Cys Ile Phe Gln Pro Ile Phe Leu Leu Ser Gly Tyr Thr Met Trp
            500                    505                    510
Ile Arg Ile Asn His Ser Leu Gly Ser Leu Asp Ser Pro Pro Thr Cys
            515                    520                    525
Val Leu Pro Asp Ser Val Val Lys Pro Leu Pro Pro Ser Ser Val Lys
            530                    535                    540
Ala Glu Ile Thr Ile Asn Ile Gly Leu Leu Lys Ile Ser Trp Glu Lys
545                    550                    555                    560
Pro Val Phe Pro Glu Asn Asn Leu Gln Phe Gln Ile Arg Tyr Gly Leu
                565                    570                    575
Ser Gly Lys Glu Val Gln Trp Lys Met Tyr Glu Val Tyr Asp Ala Lys
            580                    585                    590
Ser Lys Ser Val Ser Leu Pro Val Pro Asp Leu Cys Ala Val Tyr Ala
            595                    600                    605
Val Gln Val Arg Cys Lys Arg Leu Asp Gly Leu Gly Tyr Trp Ser Asn
            610                    615                    620
Trp Ser Asn Pro Ala Tyr Thr Val Val Met Asp Ile Lys Val Pro Met
625                    630                    635                    640
Arg Gly Pro Glu Phe Trp Arg Ile Ile Asn Gly Asp Thr Met Lys Lys
                645                    650                    655
Glu Lys Asn Val Thr Leu Leu Trp Lys Pro Leu Met Lys Asn Asp Ser
            660                    665                    670
Leu Cys Ser Val Gln Arg Tyr Val Ile Asn His His Thr Ser Cys Asn
            675                    680                    685
Gly Thr Trp Ser Glu Asp Val Gly Asn His Thr Lys Phe Thr Phe Leu
            690                    695                    700
Trp Thr Glu Gln Ala His Thr Val Thr Val Leu Ala Ile Asn Ser Ile
705                    710                    715                    720
Gly Ala Ser Val Ala Asn Phe Asn Leu Thr Phe Ser Trp Pro Met Ser
                725                    730                    735
Lys Val Asn Ile Val Gln Ser Leu Ser Ala Tyr Pro Leu Asn Ser Ser
            740                    745                    750
Cys Val Ile Val Ser Trp Ile Leu Ser Pro Ser Asp Tyr Lys Leu Met
            755                    760                    765
Tyr Phe Ile Ile Glu Trp Lys Asn Leu Asn Glu Asp Gly Glu Ile Lys
            770                    775                    780
Trp Leu Arg Ile Ser Ser Ser Val Lys Lys Tyr Tyr Ile His Asp His
785                    790                    795                    800
```

33

```
Phe Ile Pro Ile Glu Lys Tyr Gln Phe Ser Leu Tyr Pro Ile Phe Met
                805                     810                 815
Glu Gly Val Gly Lys Pro Lys Ile Ile Asn Ser Phe Thr Gln Asp Asp
                820                     825                 830
Ile Glu Lys His Gln Ser Asp
                835
```

<210> 10
<211> 820
<212> PRT
<213> Homo sapiens

<220>
<221> SOURCE
<222> 1..820
<223> /mol_type="protein" /note="Leptinrezeptor Isoform B Fragment Thr20 - Asp839" /organism="Homo sapiens"

<400> 10

```
Thr Ala Phe Asn Leu Ser Tyr Pro Ile Thr Pro Trp Arg Phe Lys Leu
1               5                   10                      15
Ser Cys Met Pro Pro Asn Ser Thr Tyr Asp Tyr Phe Leu Leu Pro Ala
            20                  25                  30
Gly Leu Ser Lys Asn Thr Ser Asn Ser Asn Gly His Tyr Glu Thr Ala
            35                  40                  45
Val Glu Pro Lys Phe Asn Ser Ser Gly Thr His Phe Ser Asn Leu Ser
    50                  55                  60
Lys Thr Thr Phe His Cys Cys Phe Arg Ser Glu Gln Asp Arg Asn Cys
65                  70                  75                      80
Ser Leu Cys Ala Asp Asn Ile Glu Gly Lys Thr Phe Val Ser Thr Val
            85                  90                  95
Asn Ser Leu Val Phe Gln Gln Ile Asp Ala Asn Trp Asn Ile Gln Cys
            100                 105                 110
Trp Leu Lys Gly Asp Leu Lys Leu Phe Ile Cys Tyr Val Glu Ser Leu
    115                 120                 125
Phe Lys Asn Leu Phe Arg Asn Tyr Asn Tyr Lys Val His Leu Leu Tyr
    130                 135                 140
Val Leu Pro Glu Val Leu Glu Asp Ser Pro Leu Val Pro Gln Lys Gly
145                 150                 155                     160
Ser Phe Gln Met Val His Cys Asn Cys Ser Val His Glu Cys Cys Glu
            165                 170                 175
Cys Leu Val Pro Val Pro Thr Ala Lys Leu Asn Asp Thr Leu Leu Met
            180                 185                 190
Cys Leu Lys Ile Thr Ser Gly Gly Val Ile Phe Gln Ser Pro Leu Met
            195                 200                 205
Ser Val Gln Pro Ile Asn Met Val Lys Pro Asp Pro Pro Leu Gly Leu
    210                 215                 220
His Met Glu Ile Thr Asp Asp Gly Asn Leu Lys Ile Ser Trp Ser Ser
225                 230                 235                     240
Pro Pro Leu Val Pro Phe Pro Leu Gln Tyr Gln Val Lys Tyr Ser Glu
            245                 250                 255
Asn Ser Thr Thr Val Ile Arg Glu Ala Asp Lys Ile Val Ser Ala Thr
            260                 265                 270
Ser Leu Leu Val Asp Ser Ile Leu Pro Gly Ser Ser Tyr Glu Val Gln
    275                 280                 285
Val Arg Gly Lys Arg Leu Asp Gly Pro Gly Ile Trp Ser Asp Trp Ser
    290                 295                 300
Thr Pro Arg Val Phe Thr Thr Gln Asp Val Ile Tyr Phe Pro Pro Lys
305                 310                 315                     320
Ile Leu Thr Ser Val Gly Ser Asn Val Ser Phe His Cys Ile Tyr Lys
            325                 330                 335
Lys Glu Asn Lys Ile Val Pro Ser Lys Glu Ile Val Trp Trp Met Asn
```

```
                    340                    345                    350
Leu Ala Glu Lys Ile Pro Gln Ser Gln Tyr Asp Val Val Ser Asp His
        355                    360                    365
Val Ser Lys Val Thr Phe Phe Asn Leu Asn Glu Thr Lys Pro Arg Gly
        370                    375                    380
Lys Phe Thr Tyr Asp Ala Val Tyr Cys Cys Asn Glu His Glu Cys His
385                    390                    395                    400
His Arg Tyr Ala Glu Leu Tyr Val Ile Asp Val Asn Ile Asn Ile Ser
                405                    410                    415
Cys Glu Thr Asp Gly Tyr Leu Thr Lys Met Thr Cys Arg Trp Ser Thr
                420                    425                    430
Ser Thr Ile Gln Ser Leu Ala Glu Ser Thr Leu Gln Leu Arg Tyr His
            435                    440                    445
Arg Ser Ser Leu Tyr Cys Ser Asp Ile Pro Ser Ile His Pro Ile Ser
        450                    455                    460
Glu Pro Lys Asp Cys Tyr Leu Gln Ser Asp Gly Phe Tyr Glu Cys Ile
465                    470                    475                    480
Phe Gln Pro Ile Phe Leu Leu Ser Gly Tyr Thr Met Trp Ile Arg Ile
                485                    490                    495
Asn His Ser Leu Gly Ser Leu Asp Ser Pro Pro Thr Cys Val Leu Pro
                500                    505                    510
Asp Ser Val Val Lys Pro Leu Pro Pro Ser Ser Val Lys Ala Glu Ile
            515                    520                    525
Thr Ile Asn Ile Gly Leu Leu Lys Ile Ser Trp Glu Lys Pro Val Phe
        530                    535                    540
Pro Glu Asn Asn Leu Gln Phe Gln Ile Arg Tyr Gly Leu Ser Gly Lys
545                    550                    555                    560
Glu Val Gln Trp Lys Met Tyr Glu Val Tyr Asp Ala Lys Ser Lys Ser
                565                    570                    575
Val Ser Leu Pro Val Pro Asp Leu Cys Ala Val Tyr Ala Val Gln Val
            580                    585                    590
Arg Cys Lys Arg Leu Asp Gly Leu Gly Tyr Trp Ser Asn Trp Ser Asn
            595                    600                    605
Pro Ala Tyr Thr Val Val Met Asp Ile Lys Val Pro Met Arg Gly Pro
    610                    615                    620
Glu Phe Trp Arg Ile Ile Asn Gly Asp Thr Met Lys Lys Glu Lys Asn
625                    630                    635                    640
Val Thr Leu Leu Trp Lys Pro Leu Met Lys Asn Asp Ser Leu Cys Ser
                645                    650                    655
Val Gln Arg Tyr Val Ile Asn His His Thr Ser Cys Asn Gly Thr Trp
            660                    665                    670
Ser Glu Asp Val Gly Asn His Thr Lys Phe Thr Phe Leu Trp Thr Glu
        675                    680                    685
Gln Ala His Thr Val Thr Val Leu Ala Ile Asn Ser Ile Gly Ala Ser
    690                    695                    700
Val Ala Asn Phe Asn Leu Thr Phe Ser Trp Pro Met Ser Lys Val Asn
705                    710                    715                    720
Ile Val Gln Ser Leu Ser Ala Tyr Pro Leu Asn Ser Ser Cys Val Ile
            725                    730                    735
Val Ser Trp Ile Leu Ser Pro Ser Asp Tyr Lys Leu Met Tyr Phe Ile
            740                    745                    750
Ile Glu Trp Lys Asn Leu Asn Glu Asp Gly Glu Ile Lys Trp Leu Arg
        755                    760                    765
Ile Ser Ser Ser Val Lys Lys Tyr Tyr Ile His Asp His Phe Ile Pro
    770                    775                    780
Ile Glu Lys Tyr Gln Phe Ser Leu Tyr Pro Ile Phe Met Glu Gly Val
785                    790                    795                    800
Gly Lys Pro Lys Ile Ile Asn Ser Phe Thr Gln Asp Asp Ile Glu Lys
                805                    810                    815
His Gln Ser Asp
            820
```

**Beschreibung**

Die vorliegende Erfindung betrifft ein In-vitro-Verfahren zum Nachweisen von mutiertem Leptin sowie die Verwendung eines Nachweisreagenzes, das nicht mutiertes Leptin, jedoch mutiertes Leptin nicht oder maximal mit 50 % des Bindungswerts von nichtmutiertem Leptin bindet, bei der Diagnose, insbesondere bei der Differenzialdiagnose, von Fettleibigkeit.

Fettleibigkeit, die auch als Adipositas oder Obesitas bezeichnet wird, ist eine Ernährungs- und Stoffwechselkrankheit, bei der die betroffenen Personen ein deutliches Übergewicht aufweisen. Das Übergewicht ist insbesondere auf das Bilden von Körperfett zurückzuführen. Hauptbestandteil des Fettgewebes sind dabei Adipozyten, wobei zwischen univakuolären Adipozyten und plurivakuolären Adipozyten unterschieden wird.

Die univakuolären Adipozyten verfügen nur über eine mit Lipiden gefüllte Vakuole. Dieser Zelltyp baut das so genannte weiße Fettgewebe auf. Bei den plurivakuolären Adipozyten werden die Lipide in mehreren voneinander getrennten Vakuolen gespeichert. Die plurivakuolären Adipozyten bauen hauptsächlich das so genannte braune Fettgewebe auf.

Die Adipozyten enthalten das Obese-Gen, welches für Leptin kodiert. In geringeren Mengen wird das Leptin auch in der Plazenta, der Magenschleimhaut, dem Knochenmark, dem Brustepithel, der Skelettmuskulatur, der Hypophyse sowie dem Hypothalamus erzeugt.

Das Leptin hemmt das Auftreten von Hungergefühlen und spielt bei der Regulierung des Fettstoffwechsels von Säugetieren, insbesondere von Menschen, eine bedeutende Rolle. Das Leptin wird dabei hauptsächlich von den Adipozyten des weißen Fettgewebes sekretiert. Der Leptinpegel ist dabei positiv mit der Körperfettmenge korreliert.

Bei dem humanen Leptin handelt es sich um ein Protein, das einschließlich Signalpeptid aus 167 Aminosäuren aufgebaut ist. Das N-terminale Signalpeptid weist eine Länge von 21 Aminosäuren auf und wird bei dem Sekretionsvorgang abgespalten, so dass das reife Leptin 146 Aminosäuren aufweist. Das Leptin wirkt durch Bindung an spezifische Leptinrezeptoren, die sowohl im Gehirn als auch in peripheren Geweben vorhanden sind. Aufgrund von alternativen Spleißvorgängen gibt es mehrere Isoformen des Leptinrezeptors. Die Isoform A ist eine kurze Leptinrezeptor-Isoform und spielt eine bedeutende Rolle beim Transport von Leptin über die Blut-Hirn-Schranke. Die Isoform B, bei der es sich um die sogenannte lange Leptin-Isoform handelt, bewirkt die Signaltransduktion und wird im Hypothalamus exprimiert.

Bei Bindung von Leptin an die Isoform B des Leptinrezeptors werden mehrere Signaltransduktionswege, einschließlich der Januskinase (JAK) und der STAT-Proteine (STAT: Signal Transducers and Activators of Transcription), insbesondere STAT-3, und der Phosphatidylinositol-3-kinase (PI3K), aktiviert. Bei anderen Signalwegen wird die Mitogen-aktivierte Proteinkinase (MAPK), 5'-Adenosin-Monophosphat-aktivierte Proteinkinase (AMPK) und das Ziel des Rapamycins im Säugetier (mTOR: mechanistic Target of Rapamycin oder auch mammalian target of rapamycin) aktiviert.

Homozygote Mutationen im Leptin-Gen führen zu einem vollständigen Leptin-Mangel, wie er in äußerst seltenen Fällen von humaner Fettleibigkeit beschrieben ist.

Die weitaus größere Mehrheit von fettleibigen Menschen weist jedoch hohe zirkulierende Leptinpegel auf (Kelesidis T. et al., Ann Intern Med. 2010, January 19, 152(2): 93-100, Narrative Review: The Role of Leptin in Human Physiology: Emerging Clinical Applications).

Patienten, die keinen nachweisbaren Leptinpegel bei Lipodystrophie, hypothalamischer Amenorrhoe und kongenitaler Leptindefizienz (CLD) aufweisen, werden mit einer Leptinersatztherapie und Gabe von Metreleptin behandelt. Bei Metreleptin handelt es sich um ein rekombinantes Leptin-Analogon, das aus 147 Aminosäuren des reifen humanen Leptins zuzüglich eines Methioninrestes am N-Terminus aufgebaut ist. Es handelt sich dabei um ein nichtglykolysiertes Polypeptid mit einer Disulfid-Bindung zwischen einer Cys-97 und Cys-147. Das Molekulargewicht beträgt etwa 16,15 kDa.

Metreleptin ist von der FDA (Food and Drug Administration, USA) zur Behandlung von Patienten, die teilweise an Lipodystrophie erkrankt sind, jedoch nicht zugelassen, weil es keine Nachweise gibt, dass bei einer heterogenen Gruppe Metreleptin sicher und wirksam ist. Metreleptin weist mithin eine sehr begrenzte Bedeutung bei der Behandlung von Patienten mit herkömmlicher Fettleibigkeit auf, da diese Patienten einen hohen Pegel an Leptin aufweisen und mithin eine zentrale Leptinresistenz zeigen (Paz-Filho G. et al., Metabolism (2014), 1-11, Leptin treatment: Facts and expectations).

Mutationen in dem für Leptin kodierenden Gen führen üblicherweise zur Abwesenheit von zirkulierendem Leptin und mithin zu extremer Fettleibigkeit. Wabitsch et al. (N Engl J Med; 372; 1; January 1, 2015; 48-54) haben bei einem zweijährigen Jungen mit extremer Fettleibigkeit eine homozygote Mutation gefunden, die zum Austausch von Guanin gegen Thymin in der Position 298 führt. In dem translatierten Leptin führt dies zu einem Aminosäureaustausch von Asparaginsäure gegen Tyrosin in der Sequenzposition 100 des Leptins (unter Berücksichtigung des Signalpeptids mit den Aminosäuren 1 bis 21). Das mutierte Protein wird sekretiert, bindet jedoch nicht an den Leptinrezeptor und aktiviert diesen auch nicht. Bei Behandlung des Patienten mit Metreleptin kam es zu einer Normalisierung des Essverhaltens und zu einem Gewichtsverlust.

Der Leptinpegel im Blut kann beispielsweise mittels RIA (Radioimmuoassay) oder ELISA (Enzyme-linked Immunoassay) bestimmt werden (Kratzsch et al., Horm Res 2002; 57: 127-132: A Rapid Quantitative Immunofunctional Assay for Measuring Human Leptin).

Lahlou et al., Diabetes, Bd. 49, August 2000, S. 1347-1352 betrifft löslichen Leptinrezeptor im Serum von Patienten mit vollständiger Resistenz gegenüber Leptin. Wu et al., J Clin Endocrinol Metab, Juni 2002, 87(6): 2931-2939 betrifft die Quantifizierung von löslichem Leptinrezeptor in humanem Blut. Fischer-Posovszky et al., J Clin Endocrinol Metab, Juni 2010, 95(6): 2836-2840 beschreibt eine Fehlsinnmutation im Leptingen, die zu einer leichten Fettleibigkeit und Hypogonadismus führt, ohne die T-Zellantwort zu beeinträchtigen. Mazen et al., Molecular Genetics and Metabolism 97(2009) 305-308 betrifft eine Fehlsinnmutation im Leptingen (N103K) bei einem fettleibigen Ägyptischen Patienten. Funcke et al., Molecular and Cellular Pediatrics 2014, 1:3 offenbart monogene Formen von Kinderfettleibigkeit aufgrund von Mutationen im Leptingen.

Aufgabe der vorliegenden Erfindung ist es, ein Verfahren bereitzustellen, das eine verbesserte Differenzialdiagnose bei Fettleibigkeit erlaubt. Des Weiteren ist es Aufgabe der Erfindung, Mittel zur Durchführung eines solchen Verfahrens bereitzustellen.

Die der Erfindung zugrunde liegende Aufgabe wird durch ein In-vitro-Verfahren zum Nachweis von mutiertem Leptin gemäß Anspruch 1 gelöst.

Das Bestimmen der Bindung von nichtmutiertem Leptin aus oder in einem ersten, flüssigen Probenmaterial, das Serum oder Plasma ist, und das Bestimmen der Bindung sowohl von mutiertem Leptin als auch von nichtmutiertem Leptin aus oder in einem zweiten, flüssigen Probenmaterial, das Serum oder Plasma ist, kann voneinander unabhängig durchgeführt werden. So kann beispielsweise das Bestimmen dieser beiden Parameter zeitlich und räumlich voneinander unabhängig erfolgen.

Das erste und das zweite flüssige Probenmaterial sind vor der Durchführung des erfindungsgemäßen Verfahrens von demselben Subjekt isoliert worden.

Das erste und das zweite Probenmaterial kann gemäß einer Variante der Erfindung aus einer Entnahme bei einem Subjekt stammen, die anschließend in zwei, drei, vier oder mehr Probenmaterialien unterteilt wurde. In diesen so erhaltenen Probenmaterialien können dann gemäß einer Variante die beiden Parameter, d.h. die Bindung von nichtmutiertem Leptin einerseits bzw. die Bindung sowohl von mutiertem Leptin als auch von nichtmutiertem Leptin andererseits, zeitlich und räumlich getrennt voneinander oder zeitlich und/oder räumlich korreliert miteinander durchgeführt werden.

Das erste und das zweite Probenmaterial können gemäß einer weiteren Variante der Erfindung einem Subjekt auch zeitlich und/oder räumlich von einander getrennt entnommen worden sein. Mithin können bei dieser Variante der Erfindung das erste und das zweite Probenmaterial aus verschiedenen Entnahmen aus einem Subjekt, beispielsweise einem Menschen, stammen.

Gemäß einer weiteren Variante der Erfindung können das erste und das zweite Probenmaterial, vor der Bestimmung der

jeweiligen Bindung, auch unterschiedlich weiter bearbeitet und/oder aufbereitet worden sein, vorzugsweise in Abhängigkeit von dem jeweils verwendeten Nachweisreagenz.

Erfindungsgemäß ist das erste Nachweisreagenz ein humaner Leptinrezeptor, der nichtmutiertes Leptin, jedoch mutiertes Leptin nicht oder mit maximal 50% des Bindungswerts von nichtmutiertem Leptin, bindet.

Erfindungsgemäß ist das zweite Nachweisreagenz ein polyklonaler oder monoklonaler Antikörper, wobei der polyklonale oder monoklonale Antikörper sowohl mutiertes als auch nichtmutiertes Leptin bindet.

Unter einem ersten bzw. zweiten Nachweisreagenz werden im Sinne der Erfindung jeweils sowohl ein einzelnes als auch eine Mehrzahl von Nachweisreagenzien, insbesondere ein oder mehrere Nachweismolekül(e), verstanden. So kann es sich bei einem Nachweisreagenz, insbesondere ein Nachweismolekül oder mehrere Nachweismoleküle, im Sinne der Erfindung, beispielsweise um eine Kombination von Nachweismolekülen wie zum Beispiel einen Erstantikörper und Zweitantikörper und/oder weiterem Antikörper und/oder Leptinrezeptormolekül(en), handeln.

Der Zweitantikörper und/oder weitere Antikörper, beispielsweise ein Tertiärantikörper, kann dabei nachweisbar markiert sein, beispielsweise mit einem Radioisotop, Fluorophor, Oligonukleotid, Biotin oder mit nachweisbaren Partikeln, beispielsweise Goldpartikeln.

Der Zweitantikörper und/oder weitere Antikörper kann auch mit einem Enzym, beispielsweise einem Reporterenzym, gekoppelt sein, so dass ein Nachweis einer Bindung durch enzymatische Umsetzung erfolgt. Beispielsweise kann es sich bei dem an einen Antikörper gekoppelten Enzym um Meerrettichperoxidase, alkalische Phosphatase, etc. handeln.

Bei einem Antikörper kann es sich um einen monoklonalen oder polyklonalen Antikörper handeln. Anstelle von Antikörpern können auch Antikörperstrukturen, wie beispielsweise die weiter unten angegebenen Antikörperfragmente oder Antikörperelemente verwendet werden.

Der Begriff "Nachweisreagenz" umfasst im Sinne der Erfindung sowohl ein einzelnes als auch mehrere Nachweisreagenzien, sofern nicht anders angegeben.

Die der Erfindung zugrunde liegende Aufgabe wird auch durch Verwendung eines humanen Leptinrezeptors, der nichtmutiertes Leptin mit einem ersten Bindungswert, jedoch mutiertes Leptin nicht oder maximal mit 50 % des Bindungswerts von nichtmutiertem Leptin bindet bzw. binden, bei der Diagnose, vorzugsweise Differenzialdiagnose, bei Fettleibigkeit gelöst.

Vorliegend wird unter einer "Zusammenstellung von Nachweisreagenzien" jede Anordnung verstanden, die sowohl das erste als auch das zweite Nachweisreagenz umfasst.

Bei dieser Anordnung handelt es sich um ein immunologisches Testsystem. Das immunologische Testsystem wird dabei aus einem RIA (Radioimmunoassay), ELISA (Enzyme-linked Immunoassay), FIA (Fluoreszenz-Immunoassay) oder LIA (Luminescence Immunoassay) ausgewählt.

Die monoklonalen Antikörper oder Antikörperfragmente können über herkömmliche Verfahren, beispielsweise die Hybridom-Technik oder die Phagen-Display-Technik, hergestellt und durch übliche Screeningverfahren bezüglich ihrer Spezifität selektiert werden.

Das erste Nachweisreagenz ist ein humaner Leptinrezeptor. Unter einem "Leptinrezeptor" wird erfindungsgemäß jede spezifisch Leptin-bindende Struktur verstanden, beispielsweise natürlich vorkommender Leptinrezeptor oder Leptinrezeptorfragmente. Auch kann es sich bei der Leptinrezeptorstruktur um synthetische oder mutierte natürlich vorkommende Leptinrezeptoren oder Leptinrezeptorfragmente handeln.

Erfindungsgemäß wesentlich ist, dass das erste Nachweisreagenz nichtmutiertes Leptin, jedoch mutiertes Leptin nicht oder nur mit maximal 50% des Bindungswertes von nichtmutiertem Leptin bindet. Vorzugsweise bindet das erste Nachweisreagenz mutiertes Leptin mit maximal 40 %, maximal 30%, maximal 20 %, maximal 10 %, maximal 5 % des Bindungswertes von nichtmutiertem Leptin. Äußerst bevorzugt bindet das erste Nachweisreagenz nichtmutiertes Leptin, jedoch mutiertes Leptin nicht oder nur mit maximal 5%, maximal 2 % des Bindungswertes von nichtmutiertem Leptin. Vorzugsweise bindet das erste Nachweisreagenz kein mutiertes Leptin.

Erfindungsgemäß ist das erste Nachweisreagenz ein humaner Leptinrezeptor. Überraschenderweise unterscheidet der humane Leptinrezeptor selektiv zwischen nichtmutiertem Leptin und mutiertem Leptin. Der humane Leptinrezeptor ist in seinen verschiedenen Isoformen für den selektiven Nachweis von nichtmutiertem Leptin in Gegenwart von mutiertem Leptin hervorragend geeignet. Anstelle des vollständigen Leptinrezeptors bzw. der verschiedenen vollständigen Isoformen können auch die entsprechenden Leptinrezeptorfragmente und/oder modifizierte Leptinrezeptoren und/oder modifizierte Leptinrezeptorfragmente verwendet werden. Beispielsweise kann die Leptinbindungsdomäne des Leptinrezeptors oder ein Fusionsprotein, das die Leptinrezeptorbindungsdomäne enthält, verwendet werden. Die Leptinrezeptoren, in fragmentierter und/oder modifizierter Form, sind durch ihre Funktionalität der selektiven Bindung von nichtmutiertem Leptin gekennzeichnet.

Erfindungsgemäß wird unter "Bindungswert" der für das jeweils verwendete Messsystem charakteristische Messwert für die Bindung von Nachweisreagenz an nichtmutiertes Leptin einerseits bzw. nichtmutiertes und mutiertes Leptin andererseits verstanden. Dieser Messwert kann entweder direkt oder indirekt ein Maß für die Bindungsaffinität sein.

Der die Bindungsaffinität reflektierende Messwert stellt dabei ein Maß für die Bindung des ersten Nachweisreagenzes an nichtmutiertes Leptin dar. Beim zweiten Nachweisreagenz stellt der die Bindungsaffinität reflektierende Messwert ein Maß für die Bindung von sowohl mutiertem als auch nichtmutiertem Leptin dar.

Der für den Bindungswert stehende Messwert kann beispielsweise die Einheit einer Konzentration (oder einer Menge) haben. So werden beispielsweise beim RIA, ELISA, FIA oder LIA üblicherweise die Konzentration (oder Menge) an bindungsfähigem nichtmutiertem Leptin einerseits oder die Konzentration (oder Menge) an bindungsfähigem nichtmutiertem und mutiertem Leptin andererseits in einer Messprobe bestimmt.

Der für den Bindungswert stehende Messwert kann auch als Gleichgewichtskonstante $K_D$, d.h. als chemisches Gleichgewicht im Sinne des Massenwirkungsgesetzes, ausgedrückt werden.

Gemäß einer Variante der Erfindung wird die Bindungsaffinität mit der Gleichgewichtskonstante $K_D$ korreliert:

$$K_D = (C_N \cdot C_L)/C_{NL,}$$

wobei:

$C_N$ die Konzentration des Nachweisreagenzes,
$C_L$ die Konzentration von (nichtmutiertem oder nichtmutiertem und mutiertem) Leptin, und
$C_{NL}$ die Konzentration des Komplexes von Nachweisreagenz und (nichtmutiertem oder nichtmutiertem und mutiertem) Leptin darstellt.

Je kleiner der $K_D$-Wert ist, umso größer ist die Bindungsaffinität.

Als Bindungswert kann auch ein anderes geeignetes Signal wie Fluoreszenz, Radioaktivitätsmessung oder ähnliches verwendet werden.

Vorzugsweise wird der Bindungswert bei der Erfindung als Konzentration ausgedrückt, so dass mithin Konzentrationen miteinander verglichen werden.

Gemäß einer Variante der Erfindung ist bevorzugt, dass der Bindungswert, d.h. der jeweils bestimmte Messwert, für die Bindung von nichtmutiertem Leptin an das erste Nachweisreagenz sowie von nichtmutiertem Leptin und mutiertem Leptin an das zweite Nachweisreagenz unter jeweils im Wesentlichen identischen, vorzugsweise unter identischen, Messbedingungen bestimmt wird.

Mithin kann die jeweilige Messung unter identischen Temperaturbedingungen, beispielsweise 20°C oder 25°C, unter im Wesentlichen identischen Pufferbedingungen, beispielsweise mit phosphatgepufferter Salzlösung pH 7,5, mit im Wesentlichen identischen Verdünnungen für einen im Wesentlichen identischen Zeitraum, beispielsweise 2 Stunden, durchgeführt werden.

Es hat sich jedoch überraschenderweise herausgestellt, dass der Nachweis der Bindung bzw. die Bestimmung des Bindungswerts auch dann mit zuverlässigem Ergebnis durchgeführt werden kann, wenn der erste Bindungswert, d.h. der bestimmte Messwert, für die Bindung von nichtmutiertem Leptin an das erste Nachweisreagenz, wobei mutiertes Leptin nicht oder maximal mit 50 % des Bindungswerts von nichtmutiertem Leptin gebunden wird, und der zweite Bindungswert der Bindung von mutiertem sowie nichtmutiertem Leptin an das zweite Nachweisreagenz unter jeweils im Wesentlichen verschiedenen, insbesondere unter verschiedenen Messbedingungen und/oder mit verschiedenen Messverfahren bestimmt wird. Bei den verschiedenen Messbedingungen kann es sich insbesondere auch um verschiedene Messverfahren handeln. Bei identischem Messverfahren können insbesondere die Bedingungen, unter denen die Messungen durchgeführt werden, voneinander abweichen.

So kann beispielsweise der Bindungswert von mutiertem und nichtmutiertem Leptin, der mithin einem Bindungswert des gesamten Leptins ("Gesamtleptin") entspricht, mit einem herkömmlichen Nachweisverfahren, beispielsweise einem affinitätsbasierten Nachweisverfahren, beispielsweise mit einem immunologischen Test wie einem ELISA, RIA, FIA, LIA, etc. in oder aus einem, vorzugsweise flüssigen, Probenmaterial eines Subjekts bestimmt werden.

Der Bindungswert von nichtmutiertem Leptin wird mit einem Nachweisverfahren bzw. mit einer Zusammenstellung von Nachweisreagenzien, beispielsweise einem Testsystem, durchgeführt, bei dem das Nachweisreagenz oder die Nachweisreagenzien zwischen mutiertem und nichtmutiertem Leptin unterscheidet/en, mithin nichtmutiertes Leptin bindet und mutiertes Leptin nicht oder mit maximal 50 % des Bindungswertes von nichtmutiertem Leptin bindet. Erfindungsgemäß wird ein humaner Leptinrezeptor oder Varianten des Leptinrezeptors verwendet. Der humane Leptinrezeptor weist überraschenderweise eine außerordentliche Spezifität für nichtmutiertes Leptin auf, bindet mithin vorzugsweise mutiertes Leptin nicht bzw. nicht nachweisbar.

Ungeachtet etwaiger möglicher Schwankungen bei der Bestimmung des Bindungswertes bei Gesamtleptin, d.h. von mutiertem und nichtmutiertem Leptin, einerseits und bei der Bestimmung des Bindungswertes von nichtmutiertem Leptin, wobei mutiertes Leptin nicht oder mit maximal 50 % des Bindungswertes von nichtmutiertem Leptin bindet, andererseits, sind die jeweiligen Bindungswerte überraschend signifikant unterschiedlich, so dass eine Differenzialdiagnose durchgeführt werden kann.

So kann beispielsweise mit der Zusammenstellung oder mit dem erfindungsgemäßen Verfahrens festgestellt werden, ob Heterozygotie oder Homozygotie bezüglich mutiertem Leptin bei einem Subjekt, vorzugsweise Mensch, vorliegt.

Das erfindungsgemäße Verfahren zum Nachweis von mutiertem Leptin wird in vitro unter Verwendung von isoliertem Probenmaterial durchgeführt. Mithin wird das erfindungsgemäße Verfahren nicht in vivo durchgeführt.

Gemäß einer bevorzugten Variante ist das zweite Nachweisreagenz ein polyklonaler Antikörper, der sowohl mutiertes Leptin als auch nichtmutiertes Leptin bindet.

Polyklonale Antikörper können auf herkömmliche Weise hergestellt werden. Beispielsweise können polyklonale Antikörper gegen humanes Leptin hergestellt werden, indem Tiere mit Leptin, optional mit Adjuvantien, immunisiert werden. Als Tiere können beispielsweise Ziege, Kaninchen, Maus oder Ratte verwendet werden. Die von den verschiedenen B-Zellen produzierten Antikörper erkennen eine Vielzahl von Epitopen auf dem Leptin, so dass sowohl mutiertes als auch nichtmutiertes Leptin von den polyklonalen Antikörpern erkannt werden.

Alternativ können auch monoklonale Antikörper verwendet werden.

Auf herkömmliche Weise werden die nach Immunisierung mit Leptin erhaltenen B-Zellen mit einer Myelomzelle fusioniert (Hybridom-Technik).

Sodann werden die erhaltenen Hybridome vereinzelt und im Hinblick auf die von den Hybridom-Zellen produzierten Antikörper in Bezug auf deren Spezifität selektioniert. Auf diese Weise können monoklonale Antikörper isoliert werden, die sowohl mutiertes als auch nichtmutiertes Leptin erkennen. Die Herstellung von monoklonalen Antikörpern und die Selektionierung von Hybridomzellen auf die Spezifität der Antikörper ist dem Fachmann bekannt.

Erfindungsgemäß ist das erste Nachweisreagenz ein humaner Leptinrezeptor. Gemäß einer weiteren Variante der Erfindung ist das erste Nachweisreagenz ein Leptinrezeptorfragment, ein modifizierter Leptinrezeptor und/oder ein modifiziertes Leptinrezeptorfragment. Beispielsweise kann es sich bei dem ersten Nachweisreagenz um die Leptinbindungsdomäne des Leptinrezeptors oder um ein Fusionsprotein, das die Leptinbindungsdomäne des Leptinrezeptors enthält, handeln. Die vorgenannten Leptinrezeptorfragmente und/oder -modifikationen weisen die Funktionalität der Selektion zwischen mutiertem und nichtmutiertem Leptin auf.

Gemäß einer weiteren bevorzugten Ausführungsform der Erfindung umfasst der Leptinrezeptor die Leptinbindungsdomäne des humanen Leptinrezeptors, vorzugsweise der Isoform A, Isoform B, Isoform C, Isoform D und/oder Isoform E.

Der Leptinrezeptor ist ein Membran-gebundener Rezeptor, der eine extrazelluläre Leptinbindungsdomäne aufweist. Die extrazelluläre Leptinbindungdomäne ist über eine Transmembranregion mit einer zytoplasmatischen Domäne verbunden.

Es existieren beim Menschen fünf Isoformen, die Isoform A, Isoform B, Isoform C, Isoform D sowie Isoform E, die alternative Spleißvarianten darstellen.

Bei der Isoform B handelt es sich um die längste Variante des Leptinrezeptors.

Die Isoform A, Isoform C sowie die Isoform D des humanen Leptinrezeptors unterscheiden sich im Hinblick auf die zytoplasmatischen Domänen. Die zytoplasmatischen Domänen sind mit der Januskinase (JAK) verbunden. Bei Bindung von Leptin an den Leptinrezeptor kommt es zur Aggregation von Rezeptorhomodimeren, wodurch eine Aktivierung der Januskinase bewirkt wird. Im Zuge der Aktivierung kommt es zur Phosphorylierung von zytoplasmatischen Transkriptionsfaktoren STAT, insbesondere von STAT-3. Die aktivierten Signaltransduktoren und Aktivatoren der Transkription (STAT) wandern zum Zellkern und bewirken die Transkription von Genen.

Die Isoformen A, C sowie D tragen nur unwesentlich zur Signaltranskription bei.

Bei der Isoform E handelt es sich um eine lösliche Form eine Leptinrezeptors, die vermutlich durch proteolytische Spaltung im Rahmen eines Rezeptor-Sheddings von der Zelloberfläche abgespalten wird.

Erfindungsgemäß wird gemäß einer bevorzugten Ausführungsform die Leptinbindungsdomäne oder die Isoform E des Leptinrezeptors als erstes Nachweisreagenz verwendet.

Die Leptinbindungsdomäne können auch als Fusionsproteine verwendet werden. So kann gemäß einer erfindungsgemäßen Variante die Leptinbindungsdomäne oder die Isoform E mit dem Fc-Teil eines Antikörpers fusioniert werden. Bei Fusionierung mit dem Fc-Teil eines Antikörpers können Homodimere der Leptinbindungsdomäne bereitgestellt werden. Diese Homodimere ähneln den bei der natürlichen Leptinbindung auf einer Zelle in vivo induzierten Homodimeren.

Gemäß einer bevorzugten Variante der Erfindung werden die Aminosäuren Thr20 bis Asp839 der Isoform B bei der Herstellung eines Fusionsproteins verwendet. Vorzugsweise werden die Aminosäuren Thr20 bis Asp839 der Isoform B mit dem Fc-Teil eines Antikörpers, vorzugsweise als Homodimer, fusioniert. Alternativ ist beispielsweise auch die Isoform E bei der Herstellung von Fusionsproteinen, beispielsweise mit dem Fc-Teil eines Antikörpers, optional als Homodimer, erfindungsgemäß geeignet.

Es hat sich überraschenderweise gezeigt, dass die Leptinbindungsdomäne des natürlichen Leptinrezeptors selektiv nichtmutiertes Leptin bindet.

Als erstes Nachweisreagenz können auch Leptinrezeptoren verwendet werden, deren Sequenz nicht identisch mit der des natürlichen Leptinrezeptors ist.

Erfindungsgemäß können mithin Leptinrezeptorvarianten des natürlichen Leptinrezeptors verwendet werden, bei denen Aminosäuren deletiert oder substituiert durch andere Aminosäuren sind, sofern nichtmutiertes Leptin nach wie vor spezifisch gebunden wird.

Gemäß einer bevorzugten Weiterbildung weist der verwendete Leptin-Rezeptor wenigstens eine Sequenzidentität von 80%, vorzugsweise von wenigstens 90%, weiter bevorzugt von wenigstens 95%, noch weiter bevorzugt von wenigstens 98%, bevorzugt von wenigstens 99%, bezogen auf die Sequenz des natürlichen Leptinrezeptors, auf.

Gemäß einer weiteren bevorzugten Ausbildung der Erfindung ist der Leptinrezeptor eine lösliche Isoform, die vorzugsweise die Aminosäuren Thr20 bis Asp839 der Isoform B des humanen Leptinrezeptors aufweist.

Gemäß einer bevorzugten Weiterbildung weist der verwendete Leptinrezeptor wenigstens eine Sequenzidentität von 80%, vorzugsweise von wenigstens 90%, weiter bevorzugt von wenigstens 95%, noch weiter bevorzugt von wenigstens 98%, noch weiter bevorzugt von wenigstens 99%, bezogen auf die Sequenz der löslichen Isoform des natürlichen Leptinrezeptors, die vorzugsweise die Aminosäuren Thr20 bis Asp839 der Isoform B aufweist, auf.

Gemäß einer weiter bevorzugten Ausführungsform der Erfindung weist das mutierte Leptin in der Aminosäuresequenz wenigstens eine der folgenden Aminosäurenaustausche D100Y, N103K und/oder L72S auf. Die Sequenzangaben beziehen bei der vorliegenden Erfindung sich dabei auf die Leptinsequenz einschließlich Signalpeptid, sofern nicht anders angegeben.

Gemäß einer weiter bevorzugten Ausführungsform der Erfindung weist das mutierte Leptin in der Aminosäuresequenz wenigstens eine der folgenden Aminosäurenaustausche R105W, G133V, S141C und/oder L161G auf.

Erfindungsgemäß weist das mutierte Leptin in der Aminosäuresequenz wenigstens eine der folgenden Aminosäurenaustausche D100Y, N103K, L72S, R105W, G133V, S141C und/oder L161G auf.

Gemäß einer weiter bevorzugten Ausführungsform der Erfindung weist das mutierte Leptin in der Aminosäuresequenz wenigstens einen Aminosäurenaustausch D100Y auf.

Bei einer bevorzugten Ausführungsform weist das mutierte Leptin in der Aminosäuresequenz den Aminosäureaustausch D100Y auf.

Bei einer bevorzugten Ausführungsform weist das mutierte Leptin in der Aminosäuresequenz den Aminosäureaustausch N103K auf.

Bei einer bevorzugten Ausführungsform weist das mutierte Leptin in der Aminosäuresequenz den Aminosäureaustausch L72S auf.

Bei einer bevorzugten Ausführungsform weist das mutierte Leptin in der Aminosäuresequenz den Aminosäureaustausch R105W auf.

Bei einer bevorzugten Ausführungsform weist das mutierte Leptin in der Aminosäuresequenz den Aminosäureaustausch G133V auf.

Bei einer bevorzugten Ausführungsform weist das mutierte Leptin in der Aminosäuresequenz den Aminosäureaustausch S141C auf.

Bei einer bevorzugten Ausführungsform weist das mutierte Leptin in der Aminosäuresequenz den Aminosäureaustausch L161G auf.

Es hat sich überraschend gezeigt, dass der natürliche Leptinrezeptor nichtmutiertes Leptin hochselektiv bindet bzw. mutiertes Leptin nicht signifikant bindet, bevorzugt nicht bindet.

So wird bereits bei einem Aminosäureaustausch im Leptin, beispielsweise an der Sequenzposition 100 von D gegen Y (D100Y), das mutierte Leptin nicht mehr von der natürlichen Bindungsdomäne des Leptinrezeptors gebunden. Bei einer äußerst bevorzugten Variante der Erfindung weist das mutierte Leptin an der Sequenzposition 100 einen Aminosäureaustausch von D gegen Y auf.

Das erfindungsgemäße Verfahren zum Nachweis von mutiertem Leptin ist in Anspruch 1 angegeben.

Das erste und das zweite flüssige Probenmaterial, das Serum oder Plasma ist, sind vor der Durchführung des erfindungsgemäßen Verfahrens, gemäß einer Variante der Erfindung zusammen, von demselben Subjekt isoliert worden. Gemäß einer weiteren Variante der Erfindung erfolgte die Entnahme des Probenmaterials bei einem Subjekt zu verschiedenen Zeitpunkten.

In Abhängigkeit von dem verwendeten Messverfahren kann die Bestimmung des ersten Bindungswertes und des zweiten Bindungswertes in einer identischen Messprobe oder separat in zwei Messproben durchgeführt werden. Im ersten Fall werden die Bindungswerte gemeinsam in einer Messprobe bestimmt, und im zweiten Fall wird das Probenmaterial in zwei Messproben aufgeteilt und separat, jedoch optional parallel, vermessen. Gemäß einer weiteren Variante der Erfindung erfolgt das Bestimmen des ersten Bindungswertes und des zweiten Bindungswertes zu verschiedenen Zeitpunkten und optional unter verschiedenen Messbedingungen und/oder optional mit verschiedenen Messverfahren.

Bei dem Subjekt handelt es sich vorzugsweise um ein Säugetier, weiter vorzugsweise um einen Menschen.

Bei dem ersten und zweiten flüssigen Probenmaterial handelt es sich um das Serum oder Plasma einer einem Subjekt, vorzugsweise einem Menschen, entnommenen Blutprobe. Nach Abnahme des Blutes kann der zelluläre Teil des Blutes vor der Gerinnung oder nach der Gerinnung abgetrennt werden. Sofern die Abtrennung vor der Gerinnung erfolgt, wird Blutplasma erhalten, erfolgt die Abtrennung nach der Gerinnung wird Blutserum erhalten.

Erfindungsgemäß kann als erstes und zweites flüssiges Probenmaterial sowohl Blutplasma als auch Blutserum verwendet werden.

Gemäß einer bevorzugten Variante der Erfindung wird Blutserum verwendet.

Die Blutprobe kann nach Abtrennung der zellulären Bestandteile getrennt werden, um ein erstes und ein zweites flüssiges Probenmaterial zu erhalten.

Nach geeigneter Verdünnung, beispielsweise in einem Puffer, beispielsweise in Phosphat-gepufferter Salzlösung, Tris-gepufferter Salzlösung oder Natriumbicarbonat-Puffer, werden das so verdünnte erste und zweite flüssige Probenmaterial mit dem ersten bzw. dem zweiten Nachweisreagenz in Kontakt gebracht und sodann der jeweilige Bindungswert bestimmt.

Der für den Bindungswert stehende Messwert kann durch verschiedene Verfahren bestimmt werden, wobei beispielsweise nach Einstellung von Gleichgewichtsbedingungen, die Konzentration an freiem Nachweisreagenz, freiem Leptin und/oder die Konzentration des Komplexes aus Nachweisreagenz und Leptin, bestimmt werden kann.

Der Bindungswert, der ein Maß für die Affinität darstellt, kann auch über einen Kompetitionsassay bestimmt werden. Der Kompetitonsassay kann beispielsweise in Form eines RIA, ELISA, FIA oder LIA auf herkömmliche Weise durchgeführt werden.

Gemäß einer Variante des erfindungsgemäßen Verfahrens kann bei der Bestimmung des Bindungswertes als Maß für die Affinität sowohl bei der Bestimmung des ersten Bindungswertes als auch bei der Bestimmung des zweiten Bindungswertes jeweils ein identisches Messverfahren unter identischen Bedingungen durchgeführt werden.

Gemäß einer weiteren Ausbildungsform des erfindungsgemäßen Verfahrens erfolgt die Bestimmung des ersten Bindungswertes und des zweiten Bindungswertes in einer einzigen Messprobe.

Bei dieser Weiterbildung der Erfindung ist keine Trennung in ein erstes und ein zweites flüssiges Probenmaterial erforderlich. Vielmehr erfolgt die Bestimmung des ersten Bindungswertes unter Verwendung des ersten Nachweisreagenzes und die Bestimmung des zweiten Bindungswertes unter Verwendung des zweiten Nachweisreagenzes in einer Messprobe, beispielsweise in einem Messvolumen. Die Messung der beiden Bindungswerte erfolgt dabei vorzugsweise gleichzeitig oder nahezu gleichzeitig.

Als Messverfahren eignen sich solche, bei denen das erste und das zweite Nachweisreagenz an partikuläre Festphasen gekoppelt sind. Diese partikulären Festphasen, an die jeweils das erste bzw. zweite Nachweisreagenz gekoppelt ist, sind beispielsweise anhand von unterschiedlichen Fluoreszenzeigenschaften, beispielsweise aufgrund der Verwendung unterschiedlichen Fluoreszenzfarbstoffe, unterscheidbar und können gemeinsam, vorzugsweise zeitgleich, beispielsweise mittels Durchflusszytometrie, vermessen werden. Diese Partikel-basierten Assays sind dem Fachmann beispielsweise als "Bead-based Multiplex-Assays" bekannt. Die Messung unter Verwendung von Partikel-basierten Multiplexverfahren können auch unter Verwendung von Biochips durchgeführt werden.

Gemäß einer äußerst bevorzugten Variante erfolgt das Bestimmen des ersten Bindungswertes und des zweiten Bindungswertes zu verschiedenen Zeitpunkten und optional unter verschiedenen Messbedingungen und/oder optional mit verschiedenen Messverfahren, wie oben ausgeführt.

Wenn der erste Bindungswert geringer ist als der zweite Bindungswert, dann ist dies ein Nachweis dafür, dass das Probenmaterial mutiertes Leptin enthält. Der Anteil an mutiertem Leptin ist sodann ein Maß für den Anteil an physiologisch nicht aktivem Leptin.

Bei der Verwendung des ersten Nachweisreagenzes wird die Bindung von nichtmutiertem Leptin gemessen. Bei der Bestimmung des zweiten Bindungswertes wird unter Verwendung des zweiten Nachweisreagenzes sowohl die Bindung von mutiertem Leptin als auch von nichtmutiertem Leptin gemessen. Der Bindungswert von mutiertem und nichtmutiertem Leptin kann auch als Bindungswert des gesamten Leptins, mithin "Gesamtleptin", bezeichnet werden.

Wenn das aus demselben Subjekt gewonnene, vorzugsweise erste und vorzugsweise zweite, flüssige Probenmaterial kein mutiertes Leptin enthält, dann ist der mit dem ersten Nachweisreagenz enthaltene erste Bindungswert identisch oder nahezu identisch zu dem unter Verwendung des zweiten Nachweisreagenzes erhaltenen zweiten Bindungswert. Als nahezu identisch wird verstanden, dass der erste und der zweite Bindungswert sich um vorzugsweise bis zu weniger als 20 %, weiter vorzugsweise um bis zu weniger als 15 %, unterscheiden.

Erfindungsgemäß wird als Verfahren ein ELISA, RIA, FIA, LIA, äußerst vorzugsweise ein ELISA, verwendet.

Gemäß einer Variante des erfindungsgemäßen Verfahrens erfolgt die Bestimmung des ersten Bindungswertes und des zweiten Bindungswertes in verschiedenen Messproben, vorzugsweise zu verschiedenen Zeitpunkten und optional mit verschiedenen Bestimmungsverfahren.

Gemäß einer weiteren Variante des erfindungsgemäßen Verfahrens erfolgt die Bestimmung des ersten Bindungswertes und des zweiten Bindungswertes mit verschiedenen Bestimmungsverfahren.

Gemäß einer weiteren Variante des erfindungsgemäßen Verfahrens liegt ein aus dem ersten und dem zweiten Bindungswert gebildeter Quotient Q:

$$Q = (\text{erster Bindungswert})/(\text{zweiter Bindungswert})$$

zwischen 0,8 und 1,2, vorzugsweise zwischen 0,9 und 1,1, oder zwischen 0,3 und 0,7, vorzugsweise zwischen 0,4 und 0,6, oder kleiner 0,2, vorzugsweise kleiner 0,1.

Gemäß einer weiteren Variante des erfindungsgemäßen Verfahrens umfasst das erste und/oder zweite Nachweisreagenz jeweils eine Kombination von zwei oder mehr Nachweisreagenzien, insbesondere von Nachweismolekülen.

Vorzugsweise eignet sich das erfindungsgemäße Verfahren zur in-vitro-Diagnose von Fettleibigkeit, wobei ein aus dem ersten und dem zweiten Bindungswert gebildeter Quotient Q:

$$Q = (\text{erster Bindungswert})/(\text{zweiter Bindungswert})$$

- zwischen 0,8 und 1,2, vorzugsweise zwischen 0,9 und 1,1, Homozygotie beim Obese-Gen in Bezug auf nichtmutiertes Leptin indiziert;
- zwischen 0,3 und 0,7, vorzugsweise zwischen 0,4 und 0,6, Heterozygotie beim Obese-Gen in Bezug auf mutiertes und nichtmutiertes Leptin indiziert; und
- kleiner 0,2, vorzugsweise bei kleiner 0,1, Homozygotie in Bezug auf mutiertes Leptin indiziert.

Gemäß einer weiteren bevorzugten Variante der Erfindung beträgt ein aus dem ersten und zweiten Bindungswert gebildeter Quotient Q:

Q = (erster Bindungswert/zweiter Bindungswert) 0,8 oder weniger.

Weiter bevorzugt beträgt der Quotient Q 0,7 oder weniger, 0,6 oder weniger, 0,5 oder weniger, 0,4 oder weniger, 0,3 oder weniger, 0,2 oder weniger oder 0,1 oder weniger.

Bei einem Wert von 0,1 oder weniger bzw. einen gegen 0 gehenden Wert ist dies ein Indiz dafür, dass der Patient homozygot bezüglich einer Leptinmutation bzw. mutiertem Leptin ist. Sofern der Quotient zwischen 0,8 und 0,1, weiter vorzugsweise zwischen 0,6 und 0,3, liegt, dann ist dies ein Indiz dafür, dass der Patient heterozygot in Bezug auf die Leptinmutation ist. In diesem Fall sekretiert der Patient sowohl nichtmutiertes als auch mutiertes Leptin.

Das Ergebnis des erfindungsgemäßen Verfahrens erlaubt eine Differenzialdiagnose bei Fettleibigkeit.

Sofern ein erheblicher Anteil an mutiertem Leptin vorliegt, ist dies im Rahmen einer Differenzialdiagnose für den Mediziner von großer Bedeutung. In diesem Fall kann, obgleich der Patient einen gegebenenfalls stark erhöhten Pegel an Leptin, wobei dieses Leptin sowohl mutiertes als auch nichtmutiertes Leptin umfasst, hat, die Verabreichung eines Leptinersatzstoffes, beispielsweise Metreleptin, angezeigt sein, um die Fettleibigkeit und gegebenenfalls weitere physiologische Störungen zu therapieren.

Sofern ein Subjekt, vorzugsweise ein Säugetier, beispielsweise ein Mensch, heterozygot bezüglich mutiertem und nichtmutiertem Leptin ist, mithin sowohl das Allel für nichtmutiertes Leptin als auch das Allel für mutiertes Leptin trägt, kann dies bei Bestimmung des Gesamtleptins nicht erkannt werden. Die vorliegende Erfindung ermöglicht jedoch eine Differenzialdiagnose, die eine Therapie insbesondere von heterozygoten Subjekten, beispielsweise mit Metreleptin, erlaubt.

Auch erlaubt die vorliegende Erfindung eine schnelle in-vitro Diagnose einer Erbkrankeit auf Proteinebene in Bezug auf mutiertes Leptin. Wenn mithin sowohl Mann und Frau heterozygot bezüglich mutiertem Leptin sind, kann Aufklärung bezüglich der Risiken einer potentiellen Fettleibigkeit etwaiger Nachkommen erfolgen. Im Rahmen einer Pränataldiagnostik kann mithin gezielt ermittelt bzw. bestätigt werden, ob bzw dass ein Risiko einer genetisch bedingten Fettleibigkeit besteht.

Die vorliegende Erfindung erlaubt mithin eine Differenzialdiagnose, die im Falle von Patienten, die nur oder auch mutiertes Leptin synthetisieren, einen neuen Therapieansatz ermöglicht.

In Abhängigkeit von dem aus dem ersten Bindungswert und zweiten Bindungswert gebildeten Quotienten Q kann des Weiteren eine Diagnose über die voraussichtliche Menge an zu verabreichendem Leptinersatzstoff, beispielsweise Metreleptin, getroffen werden.

Für die von Fettleibigkeit betroffenen Patienten, insbesondere von Kindern und Jugendlichen, stellt die vorliegende Erfindung einen wesentlichen Fortschritt bei der Diagnose, insbesondere Differenzialdiagnose, und Therapie bei Fettleibigkeit dar.

Bei dem erfindungsgemäßen Verfahren wird als das erste Nachweisreagenz ein humaner Leptinrezeptor, der nichtmutiertes Leptin, jedoch mutiertes Leptin nicht oder mit maximal 50% des Bindungswerts von nichtmutiertem Leptin, bindet, verwendet.

Bei dem erfindungsgemäßen Verfahren wird als das zweite Nachweisreagenz ein polyklonaler oder monoklonaler Antikörper, wobei der polyklonale oder monoklonale Antikörper sowohl mutiertes Leptin als auch nichtmutiertes Leptin bindet, verwendet.

Erfindungsgemäß wird als erstes Nachweisreagenz ein humaner Leptinrezeptor verwendet. Gemäß einer weiteren Variante der Erfindung wird als erstes Nachweisreagenz ein Leptinrezeptorfragment, ein modifizierter Leptinrezeptor und/oder ein modifiziertes Leptinrezeptorfragment verwendet. Beispielsweise kann es sich bei dem ersten Nachweisreagenz um die Leptinbindungsdomäne des Leptinrezeptors oder um ein Fusionsprotein, das die Leptinbindungsdomäne des Leptinrezeptors enthält, handeln. Die vorgenannten Leptinrezeptorfragmente und/oder -modifikationen weisen die Funktionalität der Selektion zwischen mutiertem und nichtmutiertem Leptin auf.

Gemäß einer weiteren bevorzugten Ausführungsform des erfindungsgemäßen Verfahrens wird bevorzugt, dass der Leptinrezeptor die Leptinbindungsdomäne des humanen Leptinrezeptors, vorzugsweise der Isoform A, Isoform B, Isoform D, umfasst.

Gemäß einer weiteren bevorzugten Weiterbildung des erfindungsgemäßen Verfahrens ist der Leptinrezeptor eine lösliche Isoform und weist vorzugsweise die Aminosäuren Thr20 bis Asp839 auf.

Weiterhin ist bevorzugt, dass das mutierte Leptin in der Aminosäuresequenz wenigstens einen der folgenden Aminosäureaustausche an den Sequenzpositionen D100Y, N103K und/oder L72S aufweist. Die Sequenzangaben beziehen sich dabei auf die Leptinsequenz einschließlich Signalpeptid.

Gemäß einer weiter bevorzugten Ausführungsform der Erfindung weist das mutierte Leptin in der Aminosäuresequenz wenigstens eine der folgenden Aminosäurenaustausche R105W, G133V, S141C und/oder L161G auf.

Erfindungsgemäß weist das mutierte Leptin in der Aminosäuresequenz wenigstens eine der folgenden Aminosäurenaustausche D100Y, N103K, L72S, R105W, G133V, S141C und/oder L161G auf.

Gemäß einer weiter bevorzugten Ausführungsform der Erfindung weist das mutierte Leptin in der Aminosäuresequenz wenigstens einen Aminosäurenaustausch D100Y auf.

Erfindungsgemäß ist das Verfahren ein immunologisches Verfahren, nämlich ein ELISA, RIA, FIA oder LIA.

Die oben im Hinblick auf die Zusammenstellung von Nachweisreagenzien gemachten Ausführungen gelten entsprechend für das erfindungsgemäße Verfahren.

Die vorliegende Erfindung betrifft ferner die Verwendung von humanem Leptinrezeptor gemäß Anspruch 10, der nichtmutiertes Leptin mit einem ersten Bindungswert bindet, jedoch mutiertes Leptin nicht oder mit maximal 50% des Bindungswertes von nichtmutiertem Leptin bindet, bei der Diagnose von Fettleibigkeit.

Vorzugsweise wird der humane Leptinrezeptor bei der Differenzialdiagnose von Fettleibigkeit verwendet.

Gemäß einer weiteren bevorzugten Ausführungsform der erfindungsgemäßen Verwendung wird der humane Leptinrezeptor, auch als erstes Nachweisreagenz bezeichnet, zusammen mit einem zweiten Nachweisreagenz verwendet. Das zweite Nachweisreagenz bindet sowohl mutiertes als auch nichtmutiertes Leptin mit einem zweiten Bindungswert.

Gemäß einer bevorzugten Weiterbildung der erfindungsgemäßen Verwendung ist das zweite Nachweisreagenz ein polyklonaler oder monoklonaler Antikörper, wobei der polyklonale oder monoklonale Antikörper sowohl mutiertes Leptin als auch nichtmutiertes Leptin bindet.

Erfindungsgemäß ist weiterhin bevorzugt, dass der als erstes Nachweisreagenz verwendete humane Leptinrezeptor die Leptinbindungsdomäne des humanen Leptinrezeptors, vorzugsweise der Isoform A, Isoform B, Isoform D, umfasst.

Gemäß einer weiteren Weiterbildung der erfindungsgemäßen Verwendung ist der als erstes Nachweisreagenz verwendete Leptinrezeptor eine lösliche Isoform und weist vorzugsweise die Aminosäuren Thr20 bis Asp839 der Isoform B auf.

Weiterhin ist bevorzugt, dass das mutierte Leptin in der Aminosäuresequenz wenigstens einen der folgenden Aminosäureaustausche an den Sequenzpositionen D100Y, N103K und/oder L72S aufweist, wobei sich die Sequenznummerierung auf die Aminosäuresequenz einschließlich Signalpeptide bezieht.

Gemäß einer bevorzugten Weiterbildung der erfindungsgemäßen Verwendung weist das mutierte Leptin einen Aminosäureaustausch D100Y in der Sequenz des natürlichen Leptins auf.

Gemäß einer bevorzugten Weiterbildung der erfindungsgemäßen Verwendung weist das mutierte Leptin einen Aminosäureaustausch N103K in der Sequenz des natürlichen Leptins auf.

Gemäß einer bevorzugten Weiterbildung der erfindungsgemäßen Verwendung weist das mutierte Leptin einen Aminosäureaustausch L72S in der Sequenz des natürlichen Leptins auf.

Vorzugsweise ist das Verfahren ein immunologisches Verfahren, weiter vorzugsweise eine ELISA, RIA, FIA oder LIA.

Die zu der oben genannten Zusammenstellung von Nachweisreagenzien bzw. zu dem erfindungsgemäßen Verfahren gemachten Ausführungen gelten für die erfindungsgemäße Verwendung entsprechend.

Die vorliegende Erfindung betrifft gemäß einer bevorzugten Ausführungsform die Verwendung von humanem Leptinrezeptor gemäß Anspruch 10, der nichtmutiertes Leptin mit einem ersten Bindungswert, jedoch mutiertes Leptin nicht oder maximal mit 50 % des Bindungswerts von nichtmutiertem Leptin bindet, bei der Diagnose von Fettleibigkeit.

Die vorliegende Erfindung betrifft gemäß einer weiteren bevorzugten Ausführungsform die erfindungsgemäße Verwendung bei der in-vitro Bestimmung von Homozygotie oder Heterozygotie in Bezug auf das für mutiertes Leptin und/oder nichtmutiertes Leptin kodierende Obese-Gen eines Säugetieres, vorzugsweise eines Menschen.

Die erfindungsgemäße Verwendung erlaubt mithin, insbesondere bei isolierten Probenmaterialien, überraschenderweise eine äußerst zuverlässige Differentialdiagnose bei Fettleibigkeit, insbesondere in Bezug auf Homozygotie und Heterozygotie bei dem für Leptin bzw. mutiertem Leptin kodierenden Obese-Gen, auf Proteinebene, insbesondere mit einem immunologischen Testsystem bzw. immunologischen Testverfahren. Immunologische Testverfahren können als Schnellteste ausgebildet sein. Insbesondere erlaubt die erfindungsgemäße Verwendung die einfache Durchführung von Massenscreenings. Sofern erwünscht, können dann für die auffälligen Proben nachfolgend aufwendige genetische Analysen zur Bestätigung durchgeführt werden.

Die nachfolgenden Figuren und Beispiele dienen der Veranschaulichung der Erfindung, ohne jedoch die Erfindung zu beschränken.

**Figuren**

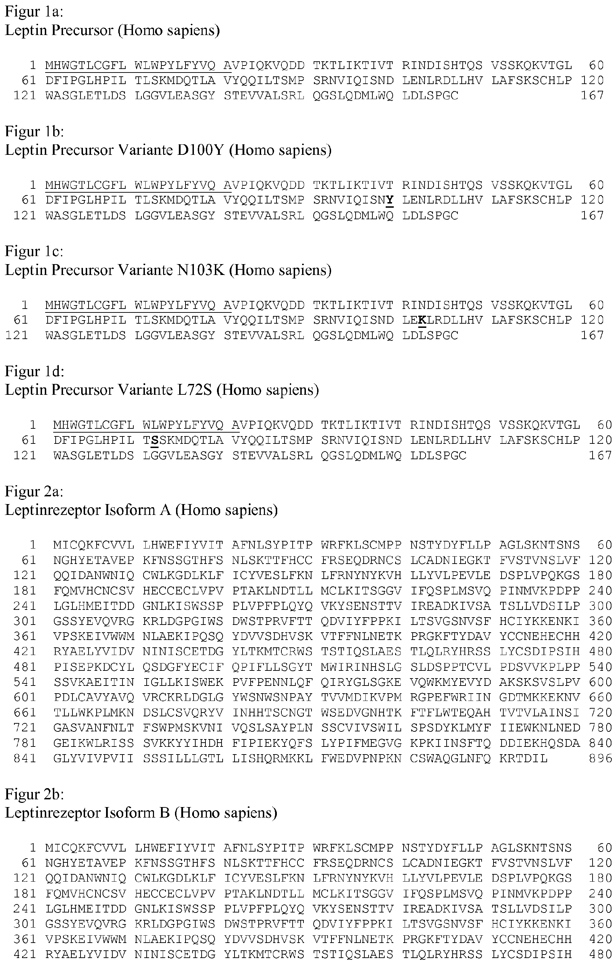

**Figur 1a** zeigt die humane Leptinvorläufersequenz, die das reife Leptin (Aminosäuren 22 bis 167) sowie das N-terminal angeordnete Signalpeptid (Aminosäuren 1 bis 21) umfasst.

**Figur 1b** zeigt die humane Leptinvorläufersequenz eines mutierten Leptins, das an der Aminosäureposition 100 einen Austausch von Asparaginsäure (D) gegen Tyrosin (Y) enthält.

**Figur 1c** zeigt die humane Leptinvorläufersequenz eines mutierten Leptins, das an der Aminosäureposition 103 einen Austausch von Asparagin (N) gegen Lysin (K) enthält.

**Figur 1d** zeigt die humane Leptinvorläufersequenz eines mutierten Leptins, das an der Aminosäureposition 72 einen Austausch von Leucin (L) gegen Serin (S) enthält.

**Figur 2a** zeigt die humane Leptinrezeptorsequenz der Isoform A.

**Figur 2b** zeigt die humane Leptinrezeptorsequenz der Isoform B.

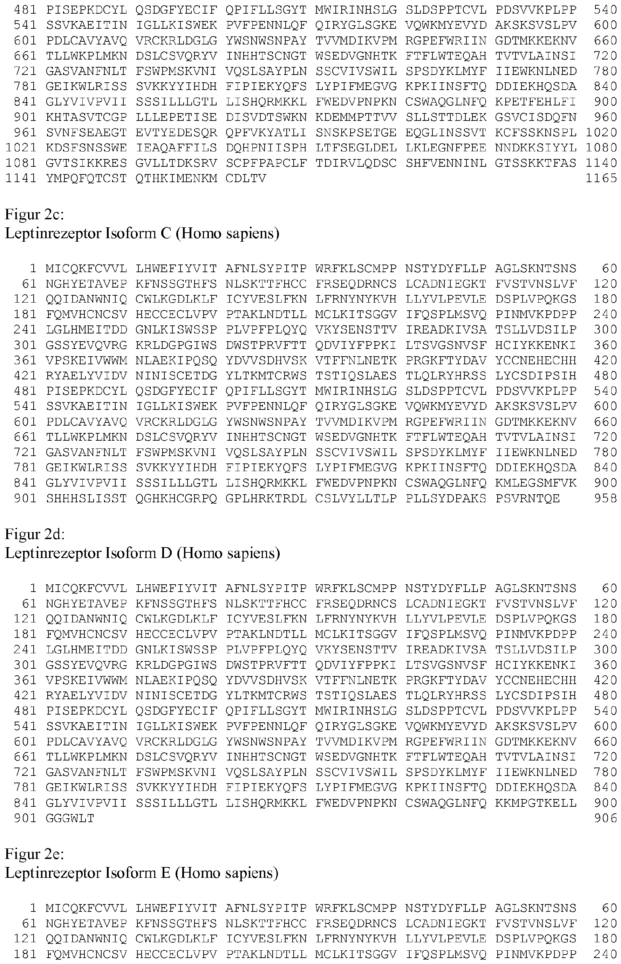

**Figur 2c** zeigt die humane Leptinrezeptorsequenz der Isoform C.

**Figur 2d** zeigt die humane Leptinrezeptorsequenz der Isoform D.

**Figur 2e** zeigt die humane Leptinrezeptorsequenz der Isoform E.

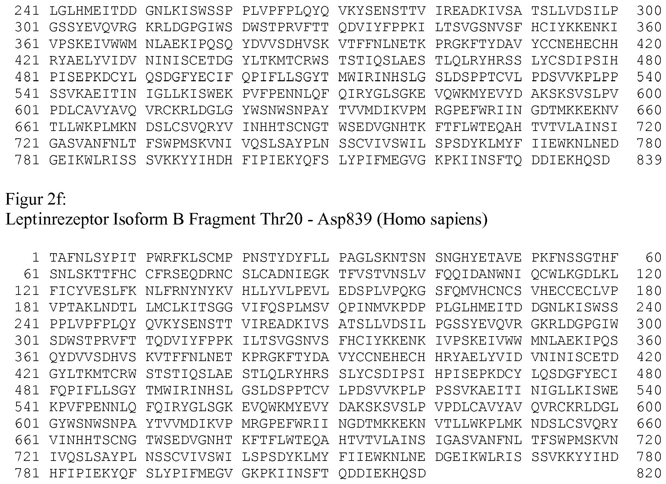

**Figur 2f** zeigt das Fragment Thr20 bis Asp839 der humanen Leptinrezeptorsequenz der Isoform B.

**Beispiele**

**Beispiel 1a: In-vitro Nachweis von Gesamt-Leptin**

Zur Herstellung eines Gesamt-Leptin ELISAs wurden handelsübliche Polystyrol Mikrotiterplatten verwendet (Fa. Greiner Bio-One GmbH, Frickenhausen, DE - Typ C8, Flat Bottom, Nr. 705071).

Adsorptiv wurde an diese Polystyrol Mikrotiterplatten als feste Phase ein kommerziell erhältlicher monoklonaler Maus-anti-humanes Leptin-IgG1 Erstantikörper in einer Konzentration von 2 μg/mL in phosphatgepufferter Salzlösung (PBS) bei pH 7,4 gebunden.

Die Platten wurden dazu über Nacht in einer feuchten Kammer gekühlt (2°C bis 8°C) inkubiert. Anschließend wurde die Antikörperlösung abgesaugt, und nicht gesättigte Bindungsstellen auf der Polystyroloberfläche mit Rinderserumalbumin (RSA) in 1%iger Lösung in PBS über 4 Stunden bei pH 7,4 abgesättigt. Die Lösung wurde abgesaugt und die Platten direkt verwendet oder bis zur Verwendung getrocknet gelagert.

Die Messungen wurden in Doppelbestimmung durchgeführt. Alle Inkubationen wurden bei Raumtemperatur (20 °C bis 25°C) durchgeführt.

1. In alle benötigten Vertiefungen der Mikrotiterplatten wurden 100 μl Verdünnungspuffer (50 mM PBS-Puffer pH 7,4 mit 0,5 % RSA und 0,05 % Detergenz Tween-20) vorgelegt.

2. In die ersten 2 Vertiefungen wurden 20 μl Verdünnungspuffer pipettiert (Leerwert). Im Anschluss daran wurden jeweils 20 μl Leptin-Standard oder 20 μl der zu messenden Probe gegeben.

3. Die Vertiefungen der Platte wurden mit Klebefolie abgedeckt und 1 Stunde bei 200 bis 350 Upm geschüttelt.

4. Nach Ablauf der Inkubationszeit wurden die Lösungen abgesaugt und die Platte mit 300 μl Waschpuffer WP (50 mM PBS-Puffer pH 7,4 mit 0,5 % RSA) / Vertiefung fünfmal gewaschen. Der Waschpuffer verblieb vor dem Absaugen etwa 15 Sekunden in den Vertiefungen.

5. Im Anschluss an den letzten Waschschritt wurden 100 μl eines Meerrettich-Peroxidase-(POD)-Konjugates eines anti-Maus-IgG1-Antikörpers in jede Vertiefung pipettiert und der Ansatz 30 Minuten bei 200 bis 350 Upm geschüttelt.

6. Nach Abschluss dieser Inkubation wurde die Platte wie in Schritt 4) beschrieben 5x gewaschen.

7. In jede Vertiefung wurden 100 μl der Substratlösung, stabilisiertes $H_2O_2$-Tetramethylbenzidin, pipettiert.

8. Die Platte wurde 15 Minuten im Dunkeln inkubiert, da das Substrat lichtempfindlich ist.

9. Nach Ablauf der Inkubationszeit wurde in jede Vertiefung 100 µl 0,1 M Schwefelsäure pipettiert.

10. Die Messung der Farbreaktion erfolgte innerhalb von 30 Minuten in einem Photometer für Mikrotiterplatten ("ELISA-Reader") bei 450 nm (Referenzfilter ≥590 nm).

Proben, die höhere Extinktionen als der Konzentrations-höchste Standard erzielten, lagen außerhalb der Standardkurve. Zur sicheren Bestimmung wurden diese Proben in einer zweiten Testdurchführung bei höherer Verdünnung nochmals gemessen.

Als Standards des ELISA wurde rekombinantes Leptin (Mediagnost, Reutlingen, DE) in einer Konzentrationen von 1, 10, 25, 50 und 100 ng/ml verwendet.

Von den Mittelwerten der optischen Dichte (OD) der Standardkonzentrationen und der Proben wurde der mittlere Leerwert OD abgezogen.

Die Standardkonzentrationen (x-Achse) wurden gegen die gemessene optische Dichte (y-Achse) aufgetragen und eine Standardkurve erstellt.

Aus der Standardkurve erhält man die Leptin-Konzentration der Kontrollen bzw. der Proben.

**Beispiel 1b: In-vitro Nachweis von funktionalem Leptin**

Für den Nachweis von funktionalem Leptin wurde der Testaufbau derart variiert, das an Polystyrol Mikrotiterplatten als feste Phase ein Leptinrezeptor gebunden wurde, der nichtmutiertes Leptin, jedoch mutiertes Leptin nicht oder maximal mit 50 % des Bindungswerts von nichtmutiertem Leptin bindet.

Als Leptinrezeptor wurde ein kommerziell erhältliches rekombinant hergestelltes Molekül mit der Aminosäuresequenz Thr20-Asp839 des humanen LeptinRezeptors verwendet (Recombinant Human Leptin R Fc Chimera, Catalog Number 389-LR/CF, R & D Systems, Minneapolis, MN, USA).

Der Leptinrezeptor wurde in einer Konzentration von 1,5 µg/mL in 10 mM Natrium-Carbonatpuffer bei pH 9,6 über Nacht in einer feuchten Kammer in gekühlter Umgebung (2°C bis 8°C) adsorptiv an Polystyrol Mikrotiterplatten gebunden.

Anschließend wurde die Beschichtungslösung abgesaugt, und nicht gesättigte Bindungsstellen auf der Polystyroloberfläche mit Rinderserumalbumin in 1%iger Lösung in PBS über 4 Stunden bei pH 7,4 abgesättigt.

Die Lösung wurde abgesaugt und die Platten direkt verwendet oder getrocknet gelagert bis zur Verwendung. Die Messungen wurden in Doppelbestimmung durchgeführt. Alle Inkubationen wurden bei Raumtemperatur (25 °C) durchgeführt.

Die zu messenden Proben bzw. Kontrollen wurden mit Verdünnungspuffer (50 mM PBS-Puffer pH 7,4 mit 0,5 % RSA und 0,05 % Detergenz Tween-20) im Verhältnis 1:10 verdünnt.

1. In alle benötigten Vertiefungen der Mikrotiterplatten wurden 100 µl Standard, bzw. 100 µl vorverdünnte Kontroll- oder Probenlösung pipettiert.

2. Die Vertiefungen der Platte wurden mit Klebefolie abgedeckt und 2 Stunden bei 350 Upm geschüttelt.

3. Nach Ablauf der Inkubationszeit wurden die Lösungen abgesaugt und die Platte mit 300 µl Waschpuffer WP / Vertiefung dreimal gewaschen. Der Waschpuffer verblieb vor dem Absaugen etwa 15 Sekunden in den Vertiefungen.

4. Im Anschluss an den letzten Waschschritt wurden 100 µl eines Biotin-Konjugates eines kommerziell erhältlichen polyklonalen Kaninchen-anti-humanes Leptin-Antikörpers in jede Vertiefung pipettiert und der Ansatz 30 Minuten bei 350 Upm geschüttelt.

5. Nach Abschluss dieser Inkubation wurde die Platte, wie in Schritt 3 beschrieben, dreimal gewaschen.

6. Im Anschluss an den letzten Waschschritt werden z.B. 100 µl eines Streptavin-Meerrettichperoxidase-Konjugates in jede Vertiefung pipettiert und der Ansatz 30 Minuten bei 350 Upm geschüttelt. Nach Abschluss dieser Inkubation wurde die Platte wie in Schritt 3) beschrieben 3x gewaschen.

7. In jede Vertiefung wurden 100 µl der Substratlösung, stabilisiertes $H_2O_2$-Tetramethylbenzidin, pipettiert.

8. Die Platte wurde 15 Minuten im Dunkeln bei 20 °C bis 25°C inkubiert, da das Substrat lichtempfindlich ist.

9. Nach Ablauf der Inkubationszeit wurde in jede Vertiefung 100 µl 0,1 M Schwefelsäure pipettiert.

10. Die Messung der Farbreaktion erfolgte innerhalb von 30 Minuten in einem Photometer für Mikrotiterplatten ("ELISA-Reader") bei 450 nm (Referenzfilter ≥590 nm).

Proben, die höhere Extinktionen als der Konzentrations-höchste Standard erzielten, lagen außerhalb der Standardkurve. Zur sicheren Bestimmung wurden diese Proben in einer zweiten Testdurchführung bei höherer Verdünnung nochmals gemessen.

Als Standards des ELISA wurde rekombinantes Leptin (Mediagnost, Reutlingen, DE) in einer Konzentrationen von 0, 0,2, 1, 2,5, 5, 7,5 und 10ng/ml verwendet.

Von den Mittelwerten der optischen Dichte (OD) der Standardkonzentrationen und der Proben wurde der mittlere Leerwert OD abgezogen.

Die Standardkonzentrationen (x-Achse) wurden gegen die gemessene optische Dichte (y-Achse) aufgetragen und eine Standardkurve erstellt.

Aus der Standardkurve erhält man die Leptin-Konzentration der verdünnten Kontrollen bzw. der Proben, und nach Multiplikation mit dem Verdünnungsfaktor dann die Leptin-Konzentration der unverdünnten Kontrollen bzw. Proben.

Nicht mutiertes funktionales Leptin wurde in diesem Test in einer Konzentration gemessen, die der im Leptin ELISA für Gesamt-Leptin gemessenen Konzentration entspricht.

**Beispiel 1c: Auswertung**

Humane Leptin cDNA (NCBI Reference Sequence: NM_000230.2) wurde verwendet um Punktmutationen durch Site-directed-mutagenesis zu erzeugen.

Dazu wurde der Q5® Site-Directed Mutagenesis Kit der Fa. New England Biolabs GmbH (Frankfurt am Main, DE) gemäß Herstellerangaben verwendet.

HEK293 Zellen wurden mit humanem Leptin (pcDNA3.1+-leptin_wt) oder mutiertem Leptin (pcDNA3.1+-leptin_D100Y oder pcDNA3.1+-leptin_N103K), wie in Sambrook et al. (Molecular cloning: a laboratory handbook, 2nd edition, 1998, Corld Spring Harbor Laboratory Press, Corld Spring Harbor, USA) beschrieben, transient transfiziert.

48 h nach der Transfektion wurden die jeweiligen Zellen lysiert und 100 µl der Überstände als Proben 1 bis 3 in den in Beispiel 1a und Beispiel 1b beschriebenen ELISA Tests verwendet.

Weiterhin wurden Serum-Proben von 5 Blutspendern sowie gereinigtes rekombinantes humanes Leptin sowie gereinigtes rekombinantes humanes Leptin Mutation D100Y verwendet.

Messergebnisse von verschiedenen Proben mit den in Beispiel 1a und Beispiel 1b beschriebenen Tests sind in Tabelle 1 dargestellt:

**Tabelle 1: Vergleich Messwerte Gesamt gegen funktionales Leptin**

| Probe Nr.: | HEK293 transient exprimierte Zellkulturüberstände | Messwerte funktionales Leptin | Messwerte Gesamt-Leptin |
|---|---|---|---|
| 1 | Leptin nicht mutiert | 490,2 ng/mL | 577,7 ng/mL |
| 2 | Leptin Mutation D100Y | < 0,5 ng/mL | 193,1 ng/mL |
| 3 | Leptin Mutation N103K | < 0.35 ng/mL | 37,1 ng/mL |
|  | **rekombinantes Leptin gereinigt** |  |  |
| 4 | Leptin nicht mutiert | 277,7 µg/mL | 335,2 µg/mL |
| 5 | Leptin Mutation D100Y | 108 ng/mL | 8,03 µg/mL |
|  | **Internationaler Standard der WHO für human Leptin WHO IS NIBSC 97/594** |  |  |
| 6 | nominal 2 ng/mL: | 1,83 ng/mL | 1,84 ng/mL |
| 7 | nominal 6 ng/mL: | 6,05 ng/mL | 6,1 ng/mL |
|  | **Serum- / Plasmaproben Blutspender** |  |  |
| 8 | **CS-2** | 2,81 ng/mL | 2,46 ng/mL |
| 9 | **DO-2226** | 4,61 ng/mL | 5,05 ng/mL |
| 10 | IDB-883- | 7,25 ng/mL | 8,31 ng/mL |
| 11 | **IDB-8960** | 23,96 ng/mL | 25,05 ng/mL |
| 12 | **BB-3935** | 34,12 ng/mL | 34,84 ng/mL |

Tabelle 1 ist zu entnehmen, dass mit der erfindungsgemäßen Zusammenstellung bzw. dem erfindungsgemäßen Verfahren zwischen mutiertem Leptin und nichtmutiertem Leptin unterschieden werden kann.

Probe Nr. 1 enthält ausschließlich nichtmutiertes Leptin. Der Quotient der Konzentration von nichtmutiertem Leptin (funktionalem Leptin) und Gesamtleptin beträgt etwa 0,85 (490,2/577,7).

Probe Nr.2 enthält ausschließlich mutiertes Leptin mit der Mutation D100Y. Der Quotient der Konzentration von nichtmutiertem Leptin (funktionalem Leptin) und Gesamtleptin beträgt < 0,01 (0,5/193,1).

Probe Nr.3 enthält ausschließlich mutiertes Leptin mit der Mutation N103K. Der Quotient der Konzentration von nichtmutiertem Leptin (funktionalem Leptin) und Gesamtleptin beträgt < 0,01 (0,35/37,1).

Die Proben Nr. 4 und 5 enthalten humanes Leption in einer Konzentration von 2 ng/ml bzw. 6 ng/ml gemäß WHO-Standard. Der Quotient der Konzentration von nichtmutiertem Leptin (funktionalem Leptin) und Gesamtleptin beträgt 0,99 (1,83/1,84) bzw. 0,99 (6,05/6,1).

Die Proben Nr. 8 bis 12 sind Serum-/Plasmaproben von gesunden Blutspendern. Der Quotient der Konzentration von nichtmutiertem Leptin (funktionalem Leptin) und Gesamtleptin beträgt 1,14 (2,81/2,46), 0,91 (4,61/5,05), 0,87 (7,25/8,31), 0,96 (23,96/25,05) bzw. 0,98 (34,12/34,84).

**Beispiel 2**

Bei einem adipösen Kleinkind (nicht in Tabelle 1 gezeigt) wurden in Serum-/Plasmaproben eine Gesamtleptinkonzentration von 30,1 ng/ml und eine Konzentration an nichtmutiertem Leptin (funktionalem Leptin) von 0,3 ng/ml bestimmt. Der Quotient der Konzentration von nichtmutiertem Leptin (funktionalem Leptin) und Gesamtleptin beträgt mithin 0,01 (0,3/30,1).
Sodann wurden bei Vater und Mutter die Konzentration im Blutplasma/-serum an nichtmutiertem Leptin (funktionalem Leptin) bzw. Gesamtleptin bestimmt:
Beim Vater wurden in Serum-/Plasmaproben eine Gesamtleptinkonzentration von 2,3 ng/ml und eine Konzentration an nichtmutiertem Leptin (funktionalem Leptin) von 1,2 ng/ml bestimmt. Der Quotient der Konzentration von nichtmutiertem Leptin (funktionalem Leptin) und Gesamtleptin beträgt mithin 0,52 (1,2/2,3).
Bei der Mutter wurden in Serum-/Plasmaproben eine Gesamtleptinkonzentration von 11,4 ng/ml und eine Konzentration an nichtmutiertem Leptin (funktionalem Leptin) von 4,8 ng/ml bestimmt. Der Quotient der Konzentration von nichtmutiertem Leptin (funktionalem Leptin) und Gesamtleptin beträgt mithin 0,42 (4,8/11,4).
Aus den gemessen Konzentrationswerten von nichtmutiertem Leptin und Gesamtleptin bzw. den daraus berechneten Quotienten lässt sich unmittelbar erkennen, dass Vater und Mutter heterozygot in Bezug auf mutiertes Leptin bzw. nichtmutiertes Leptin sind.
Das Kind von Vater und Mutter ist jedoch homozygot in Bezug auf mutiertes Leptin.
Bei dem mutierten Leptin von Vater, Mutter und Kind handelte es sich um Leptin mit einer D100Y-Mutation.
Die erfindungsgemäße Zusammenstellung und/oder das erfindungsgemäße Verfahren erlauben mithin überraschenderweise eine äußerst zuverlässige Differentialdiagnose bei Fettleibigkeit, insbesondere in Bezug auf Homozygotie und Heterozygotie bei dem für Leptin bzw. mutiertem Leptin kodierenden Obese-Gen, auf Proteinebene, insbesondere mit einem immunologischen Testsystem bzw. immunologischen Testverfahren. Immunologische Testverfahren können als Schnellteste ausgebildet sein. Insbesondere ist das erfindungsgemäße Verfahren wesentlich schneller, auch im Rahmen einer Massenscreenings, durchzuführen als genetische Analysen.

**Patentansprüche**

1. In-vitro-Verfahren zum Nachweis von mutiertem Leptin,
   **dadurch gekennzeichnet,**
   **dass** das Verfahren folgende Schritte umfasst:

   - Bestimmen der Bindung von nichtmutiertem Leptin aus Serum oder Plasma an humanem Leptinrezeptor, der nichtmutiertes Leptin, jedoch mutiertes Leptin nicht oder maximal mit 50 % des Bindungswerts von nichtmutiertem Leptin unter Erhalt eines ersten Bindungswerts bindet, wobei das mutierte Leptin in der Aminosäuresequenz wenigstens einen der folgenden Aminosäureaustausche D100Y, N103K, L72S, R105W, G133V, S141C und/oder L161G, vorzugsweise D100Y, N103K und/oder L72S, weiter vorzugsweise D100Y, aufweist
   - Bestimmen der Bindung sowohl von mutiertem Leptin als auch von nichtmutiertem Leptin aus Serum oder Plasma an einen polyklonalen oder monoklonalen Antikörper, wobei der polyklonale oder monoklonale Antikörper sowohl mutiertes Leptin als auch nichtmutiertes Leptin bindet, unter Erhalt eines zweiten Bindungswerts,

   wobei mutiertes Leptin vorhanden ist, wenn der erste Bindungswert kleiner als der zweite Bindungswert ist, und wobei die Bindungswerte mittels ELISA,RIA, FIA oder LIA bestimmt werden.

2. Verfahren nach Anspruch 1,
   **dadurch gekennzeichnet,**
   **dass** der humane Leptinrezeptor die Isoform A, Isoform B, Isoform C, Isoform D und/oder Isoform E, umfasst.

3. Verfahren nach Anspruch 1 oder 2,
   **dadurch gekennzeichnet,**
   **dass** der Leptinrezeptor eine lösliche Isoform ist und vorzugsweise die Aminosäuren Thr20 bis Asp839 der Isoform B des humanen Leptinrezeptors aufweist.

4. Verfahren nach einem der Ansprüche 1 bis 3,
   **dadurch gekennzeichnet,**
   **dass** die Bestimmung des ersten Bindungswertes und des zweiten Bindungswertes in einer Messprobe erfolgt.

5. Verfahren nach einem der Ansprüche 1 bis 4,
   **dadurch gekennzeichnet,**
   **dass** die Bestimmung des ersten Bindungswertes und des zweiten Bindungswertes in verschiedenen Messproben, vorzugsweise zu verschiedenen Zeitpunkten und optional mit verschiedenen Bestimmungsverfahren, erfolgt.

6. Verfahren nach einem der Ansprüche 1 bis 5,
   **dadurch gekennzeichnet,**
   **dass** die Bestimmung des ersten Bindungswertes und des zweiten Bindungswertes mit verschiedenen Bestimmungsverfahren erfolgt.

7. Verfahren nach einem der Ansprüche 1 bis 6,
   **dadurch gekennzeichnet,**
   **dass** ein aus dem ersten und dem zweiten Bindungswert gebildeter Quotient Q:

$$Q = (\text{erster Bindungswert})/(\text{zweiter Bindungswert})$$

   zwischen 0,8 und 1,2, vorzugsweise zwischen 0,9 und 1,1, oder
   zwischen 0,3 und 0,7, vorzugsweise zwischen 0,4 und 0,6, oder
   kleiner 0,2, vorzugsweise kleiner 0,1,
   liegt.

8. Verfahren nach einem der Ansprüche 1 bis 7,
   **dadurch gekennzeichnet,**
   **dass** ein aus dem ersten und dem zweiten Bindungswert gebildeter Quotient Q:

$$Q = (\text{erster Bindungswert})/(\text{zweiter Bindungswert})$$

0,8 oder weniger beträgt.

9. Verfahren nach einem der Ansprüche 1 bis 8,
**dadurch gekennzeichnet,**
zur in-vitro-Diagnose von Fettleibigkeit, wobei ein aus dem ersten und dem zweiten Bindungswert gebildeter Quotient Q:

$$Q = (\text{erster Bindungswert})/(\text{zweiter Bindungswert})$$

- zwischen 0,8 und 1,2, vorzugsweise zwischen 0,9 und 1,1, Homozygotie beim Obese-Gen in Bezug auf nichtmutiertes Leptin indiziert;
- zwischen 0,3 und 0,7, vorzugsweise zwischen 0,4 und 0,6, Heterozygotie beim Obese-Gen in Bezug auf mutiertes und nichtmutiertes Leptin indiziert; und
- kleiner 0,2, vorzugsweise bei kleiner 0,1, Homozygotie in Bezug auf mutiertes Leptin indiziert,

10. Verwendung von humanem Leptinrezeptor, der nichtmutiertes Leptin mit einem ersten Bindungswert, jedoch mutiertes Leptin nicht oder maximal mit 50 % des Bindungswerts von nichtmutiertem Leptin bindet, bei der in-vitro Diagnose von Fettleibigkeit, wobei das mutierte Leptin in der Aminosäuresequenz wenigstens einen der folgenden Aminosäureaustausche D100Y, N103K, L72S, R105W, G133V, S141C und/oder L161G, vorzugsweise D100Y, N103K und/oder L72S, weiter vorzugsweise D100Y, aufweist.

11. Verwendung nach Anspruch 10 bei der in-vitro Bestimmung von Homozygotie oder Heterozygotie in Bezug auf das für mutiertes Leptin und/oder nichtmutiertes Leptin kodierende Obese-Gen eines Säugetieres, vorzugsweise eines Menschen.

12. Verwendung nach Anspruch 10 oder 11, wobei der humane Leptinrezeptor die Isoform A, Isoform B, Isoform C, Isoform D und/oder Isoform E, umfasst.

13. Verwendung nach einen der Ansprüche 10 bis 12, wobei der Leptinrezeptor eine lösliche Isoform ist und vorzugsweise die Aminosäuren Thr20 bis Asp839 der Isoform B des humanen Leptinrezeptors aufweist.

**Claims**

1. In-vitro method for detecting mutated leptin,
**characterised in that**
the method comprises the following steps:

- determining the binding of non-mutated leptin made of serum or plasma to the human leptin receptor, which binds non-mutated leptin but does not bind mutated leptin, or binds it with a maximum of 50% of the binding value of non-mutated leptin, by obtaining a first binding value, wherein, in the amino acid sequence, the mutated leptin has at least one of the following amino acid exchanges: D100Y, N103K, L72S, R105W, G133V, S141C and/or L161G, preferably D100Y, N103K and/or L72S, more preferably D100Y,
- determining the binding of both mutated leptin and non-mutated leptin made of serum or plasma to a polyclonal or monoclonal antibody, wherein the polyclonal or monoclonal antibody binds both mutated leptin and non-mutated leptin, by obtaining a second binding value,

wherein mutated leptin is present when the first binding value is smaller than the second binding value,
and wherein the binding values are determined by means of ELISA, RIA, FIA or LIA.

2. Method according to claim 1,
**characterised in that**
the human leptin receptor comprises isoform A, isoform B, isoform C, isoform D and/or isoform E.

**3.** Method according to claim 1 or claim 2,
**characterised in that**
the leptin receptor is a soluble isoform and preferably has the amino acids Thr20 to Asp839 of isoform B of the human leptin receptor.

**4.** Method according to one of claims 1 to 3,
**characterised in that**
the first binding value and the second binding value are determined in one test sample.

**5.** Method according to one of claims 1 to 4,
**characterised in that**
the first binding value and the second binding value are determined in different test samples, preferably at different points in time, and optionally using different determination methods.

**6.** Method according to one of claims 1 to 5,
**characterised in that**
the first binding value and the second binding value are determined using different determination methods.

**7.** Method according to one of claims 1 to 6,
**characterised in that**
a quotient Q obtained from the first and the second binding value:

$$Q = (\text{first binding value})/(\text{second binding value})$$

is between 0.8 and 1.2, preferably between 0.9 and 1.1, or
is between 0.3 and 0.7, preferably between 0.4 and 0.6, or
is less than 0.2, preferably less than 0.1.

**8.** Method according to one of claims 1 to 7,
**characterised in that**
a quotient Q obtained from the first and the second binding value:

$$Q = (\text{first binding value})/(\text{second binding value})$$

is 0.8 or less.

**9.** Method according to one of claims 1 to 8,
**characterised in that,**
for the in-vitro diagnosis of obesity, a quotient Q obtained from the first and the second binding value:

$$Q = (\text{first binding value})/(\text{second binding value})$$

- between 0.8 and 1.2, preferably between 0.9 and 1.1, indicates homozygosity in the obesity gene with respect to non-mutated leptin;
- between 0.3 and 0.7, preferably between 0.4 and 0.6, indicates heterozygosity in the obesity gene with respect to mutated and non-mutated leptin; and
- less than 0.2, preferably less than 0.1, indicates homozygosity with respect to mutated leptin.

**10.** Use of the human leptin receptor, which binds non-mutated leptin with a first binding value but does not bind mutated leptin, or binds it with a maximum of 50 % of the binding value of non-mutated leptin, in the in-vitro diagnosis of obesity, wherein, in the amino acid sequence, the mutated leptin has at least one of the following amino acid exchanges: D100Y, N103K, L72S, R105W, G133V, S141C and/or L161G, preferably D100Y, N103K and/or L72S, more preferably D100Y.

**11.** Use according to claim 10 in the in-vitro determination of homozygosity or heterozygosity with respect to the obesity gene of a mammal, preferably a human, which encodes mutated leptin and/or non-mutated leptin.

**12.** Use according to claim 10 or claim 11, wherein the human leptin receptor comprises isoform A, isoform B, isoform C, isoform D and/or isoform E.

**13.** Use according to one of claims 10 to 12, wherein the leptin receptor is a soluble isoform and preferably has the amino acids Thr20 to Asp839 of isoform B of the human leptin receptor.

**Revendications**

**1.** Procédé in vitro pour la détection de leptine mutée, **caractérisé en ce qu'**il comprend les étapes suivantes :

- détermination de la liaison de leptine non mutée provenant du sérum ou du plasma au récepteur de leptine humaine, qui se lie à la leptine non mutée, mais ne se lie pas à la leptine mutée, ou se lie au maximum à 50 % de la valeur de liaison de la leptine non mutée, avec obtention d'une première valeur de liaison, la leptine mutée présentant, dans sa séquence d'acides aminés, au moins l'un des échanges d'acides aminés suivants : D100Y, N103K, L72S, R105W, G133V, S141C et/ou L161G, de préférence D100Y, N103K et/ou L72S, plus préférentiellement D100Y,
- détermination de la liaison tant de la leptine mutée que de la leptine non mutée provenant du sérum ou du plasma à un anticorps polyclonal ou monoclonal, l'anticorps polyclonal ou monoclonal se liant tant à la leptine mutée qu'à la leptine non mutée, avec obtention d'une deuxième valeur de liaison,

la leptine mutée étant présente quand la première valeur de liaison est inférieure à la deuxième valeur de liaison, et les valeurs de liaison étant déterminées par ELISA, RIA, FIA ou LIA.

**2.** Procédé selon la revendication 1, **caractérisé en ce que** le récepteur de leptine humaine comprend l'isoforme A, l'isoforme B, l'isoforme C, l'isoforme D et/ou l'isoforme E.

**3.** Procédé selon la revendication 1 ou 2, **caractérisé en ce que** le récepteur de leptine est une isoforme soluble, et de préférence présente les acides aminés Thr20 à Asp839 de l'isoforme B du récepteur de leptine humaine.

**4.** Procédé selon l'une des revendications 1 à 3, **caractérisé en ce que** la détermination de la première valeur de liaison et de la deuxième valeur de liaison a lieu dans un échantillon de mesure.

**5.** Procédé selon l'une des revendications 1 à 4, **caractérisé en ce que** la détermination de la première valeur de liaison et de la deuxième valeur de liaison a lieu dans différents échantillons d'essai, de préférence à des instants différents et en option par des procédés de détermination différents.

**6.** Procédé selon l'une des revendications 1 à 5, **caractérisé en ce que** la détermination de la première valeur de liaison et de la deuxième valeur de liaison a lieu avec des procédés de détermination différents.

**7.** Procédé selon l'une des revendications 1 à 6, **caractérisé en ce qu'**un quotient Q, formé à partir de la première et de la deuxième valeurs de liaison :

$$Q = \text{(première valeur de liaison)}/\text{(deuxième valeur de liaison)}$$

est compris entre 0,8 et 1,2, de préférence entre 0,9 et 1,1, ou entre 0,3 et 0,7, de préférence entre 0,4 et 0,6, ou est inférieur à 0,2, de préférence inférieur à 0,1.

**8.** Procédé selon l'une des revendications 1 à 7, **caractérisé en ce qu'**un quotient Q formé à partir de la première et de la deuxième valeurs de liaison :

$$Q = \text{(première valeur de liaison)}/\text{(deuxième valeur de liaison)}$$

est de 0,8 ou moins.

9.  Procédé selon l'une des revendications 1 à 8, **caractérisé en ce que**, pour le diagnostic in vitro de l'obésité, un quotient Q formé à partir de la première et de la deuxième valeurs de liaison :

$$Q = (première\ valeur\ de\ liaison)/(deuxième\ valeur\ de\ liaison)$$

- entre 0,8 et 1,2, de préférence entre 0,9 et 1,1, indique une homozygotie dans le gène de l'obésité pour la leptine non mutée ;
- entre 0,3 et 0,7, de préférence entre 0,4 et 0,6, indique une hétérozygotie dans le gène de l'obésité pour la leptine mutée et la leptine non mutée ; et
- inférieur à 0,2, de préférence inférieur à 0,1, une homozygotie pour la leptine mutée.

10. Utilisation du récepteur de leptine humaine, qui se lie à la leptine non mutée avec une première valeur de liaison, mais ne se lie pas à la leptine mutée ou au maximum avec 50 % de la valeur de liaison de la leptine non mutée, pour le diagnostic in vitro de l'obésité, la leptine mutée présentant dans sa séquence d'acides aminés au moins l'un des échanges d'acides aminés suivant : D100Y, N103K, L72S, R105W, G133V, S141C et/ou L161G, de préférence D100Y, N103K et/ou L72S.

11. Utilisation selon la revendication 10 pour la détermination in vitro d'une homozygotie ou d'une hétérozygotie, en liaison avec le gène de l'obésité codant pour la leptine mutée et/ou la leptine non mutée d'un mammifère, de préférence l'homme.

12. Utilisation selon la revendication 10 ou 11, pour laquelle le récepteur de leptine humaine comprend l'isoforme A, l'isoforme B, l'isoforme C, l'isoforme D et/ou l'isoforme E.

13. Utilisation selon l'une des revendications 10 à 12, pour laquelle le récepteur de leptine est une isoforme soluble et présente de préférence les acides aminés Thr20 à Asp839 de l'isoforme B du récepteur de leptine humaine.

Figur 1a:
Leptin Precursor (Homo sapiens)

```
  1 MHWGTLCGFL WLWPYLFYVQ AVPIQKVQDD TKTLIKTIVT RINDISHTQS VSSKQKVTGL  60
 61 DFIPGLHPIL TLSKMDQTLA VYQQILTSMP SRNVIQISND LENLRDLLHV LAFSKSCHLP 120
121 WASGLETLDS LGGVLEASGY STEVVALSRL QGSLQDMLWQ LDLSPGC            167
```

Figur 1b:
Leptin Precursor Variante D100Y (Homo sapiens)

```
  1 MHWGTLCGFL WLWPYLFYVQ AVPIQKVQDD TKTLIKTIVT RINDISHTQS VSSKQKVTGL  60
 61 DFIPGLHPIL TLSKMDQTLA VYQQILTSMP SRNVIQISNY LENLRDLLHV LAFSKSCHLP 120
121 WASGLETLDS LGGVLEASGY STEVVALSRL QGSLQDMLWQ LDLSPGC            167
```

Figur 1c:
Leptin Precursor Variante N103K (Homo sapiens)

```
  1 MHWGTLCGFL WLWPYLFYVQ AVPIQKVQDD TKTLIKTIVT RINDISHTQS VSSKQKVTGL  60
 61 DFIPGLHPIL TLSKMDQTLA VYQQILTSMP SRNVIQISND LEKLRDLLHV LAFSKSCHLP 120
121 WASGLETLDS LGGVLEASGY STEVVALSRL QGSLQDMLWQ LDLSPGC            167
```

Figur 1d:
Leptin Precursor Variante L72S (Homo sapiens)

```
  1 MHWGTLCGFL WLWPYLFYVQ AVPIQKVQDD TKTLIKTIVT RINDISHTQS VSSKQKVTGL  60
 61 DFIPGLHPIL TSSKMDQTLA VYQQILTSMP SRNVIQISND LENLRDLLHV LAFSKSCHLP 120
121 WASGLETLDS LGGVLEASGY STEVVALSRL QGSLQDMLWQ LDLSPGC            167
```

Figur 2a:
Leptinrezeptor Isoform A (Homo sapiens)

```
  1 MICQKFCVVL LHWEFIYVIT AFNLSYPITP WRFKLSCMPP NSTYDYFLLP AGLSKNTSNS  60
 61 NGHYETAVEP KFNSSGTHFS NLSKTTFHCC FRSEQDRNCS LCADNIEGKT FVSTVNSLVF 120
121 QQIDANWNIQ CWLKGDLKLF ICYVESLFKN LFRNYNYKVH LLYVLPEVLE DSPLVPQKGS 180
181 FQMVHCNCSV HECCECLVPV PTAKLNDTLL MCLKITSGGV IFQSPLMSVQ PINMVKPDPP 240
241 LGLHMEITDD GNLKISWSSP PLVPFPLQYQ VKYSENSTTV IREADKIVSA TSLLVDSILP 300
301 GSSYEVQVRG KRLDGPGIWS DWSTPRVFTT QDVIYFPPKI LTSVGSNVSF HCIYKKENKI 360
361 VPSKEIVWWM NLAEKIPQSQ YDVVSDHVSK VTFFNLNETK PRGKFTYDAV YCCNEHECHH 420
421 RYAELYVIDV NINISCETDG YLTKMTCRWS TSTIQSLAES TLQLRYHRSS LYCSDIPSIH 480
481 PISEPKDCYL QSDGFYECIF QPIFLLSGYT MWIRINHSLG SLDSPPTCVL PDSVVKPLPP 540
541 SSVKAEITIN IGLLKISWEK PVFPENNLQF QIRYGLSGKE VQWKMYEVYD AKSKSVSLPV 600
601 PDLCAVYAVQ VRCKRLDGLG YWSNWSNPAY TVVMDIKVPM RGPEFWRIIN GDTMKKEKNV 660
661 TLLWKPLMKN DSLCSVQRYV INHHTSCNGT WSEDVGNHTK FTFLWTEQAH TVTVLAINSI 720
721 GASVANFNLT FSWPMSKVNI VQSLSAYPLN SSCVIVSWIL SPSDYKLMYF IIEWKNLNED 780
781 GEIKWLRISS SVKKYYIHDH FIPIEKYQFS LYPIFMEGVG KPKIINSFTQ DDIEKHQSDA 840
841 GLYVIVPVII SSSILLLGTL LISHQRMKKL FWEDVPNPKN CSWAQGLNFQ KRTDIL     896
```

Figur 2b:
Leptinrezeptor Isoform B (Homo sapiens)

```
  1 MICQKFCVVL LHWEFIYVIT AFNLSYPITP WRFKLSCMPP NSTYDYFLLP AGLSKNTSNS  60
 61 NGHYETAVEP KFNSSGTHFS NLSKTTFHCC FRSEQDRNCS LCADNIEGKT FVSTVNSLVF 120
121 QQIDANWNIQ CWLKGDLKLF ICYVESLFKN LFRNYNYKVH LLYVLPEVLE DSPLVPQKGS 180
181 FQMVHCNCSV HECCECLVPV PTAKLNDTLL MCLKITSGGV IFQSPLMSVQ PINMVKPDPP 240
241 LGLHMEITDD GNLKISWSSP PLVPFPLQYQ VKYSENSTTV IREADKIVSA TSLLVDSILP 300
301 GSSYEVQVRG KRLDGPGIWS DWSTPRVFTT QDVIYFPPKI LTSVGSNVSF HCIYKKENKI 360
361 VPSKEIVWWM NLAEKIPQSQ YDVVSDHVSK VTFFNLNETK PRGKFTYDAV YCCNEHECHH 420
421 RYAELYVIDV NINISCETDG YLTKMTCRWS TSTIQSLAES TLQLRYHRSS LYCSDIPSIH 480
```

```
 481 PISEPKDCYL QSDGFYECIF QPIFLLSGYT MWIRINHSLG SLDSPPTCVL PDSVVKPLPP  540
 541 SSVKAEITIN IGLLKISWEK PVFPENNLQF QIRYGLSGKE VQWKMYEVYD AKSKSVSLPV  600
 601 PDLCAVYAVQ VRCKRLDGLG YWSNWSNPAY TVVMDIKVPM RGPEFWRIIN GDTMKKEKNV  660
 661 TLLWKPLMKN DSLCSVQRYV INHHTSCNGT WSEDVGNHTK FTFLWTEQAH TVTVLAINSI  720
 721 GASVANFNLT FSWPMSKVNI VQSLSAYPLN SSCVIVSWIL SPSDYKLMYF IIEWKNLNED  780
 781 GEIKWLRISS SVKKYYIHDH FIPIEKYQFS LYPIFMEGVG KPKIINSFTQ DDIEKHQSDA  840
 841 GLYVIVPVII SSSILLLGTL LISHQRMKKL FWEDVPNPKN CSWAQGLNFQ KPETFEHLFI  900
 901 KHTASVTCGP LLLEPETISE DISVDTSWKN KDEMMPTTVV SLLSTTDLEK GSVCISDQFN  960
 961 SVNFSEAEGT EVTYEDESQR QPFVKYATLI SNSKPSETGE EQGLINSSVT KCFSSKNSPL 1020
1021 KDSFSNSSWE IEAQAFFILS DQHPNIISPH LTFSEGLDEL LKLEGNFPEE NNDKKSIYYL 1080
1081 GVTSIKKRES GVLLTDKSRV SCPFPAPCLF TDIRVLQDSC SHFVENNINL GTSSKKTFAS 1140
1141 YMPQFQTCST QTHKIMENKM CDLTV                                       1165
```

Figur 2c:
Leptinrezeptor Isoform C (Homo sapiens)

```
   1 MICQKFCVVL LHWEFIYVIT AFNLSYPITP WRFKLSCMPP NSTYDYFLLP AGLSKNTSNS   60
  61 NGHYETAVEP KFNSSGTHFS NLSKTTFHCC FRSEQDRNCS LCADNIEGKT FVSTVNSLVF  120
 121 QQIDANWNIQ CWLKGDLKLF ICYVESLFKN LFRNYNYKVH LLYVLPEVLE DSPLVPQKGS  180
 181 FQMVHCNCSV HECCECLVPV PTAKLNDTLL MCLKITSGGV IFQSPLMSVQ PINMVKPDPP  240
 241 LGLHMEITDD GNLKISWSSP PLVPFPLQYQ VKYSENSTTV IREADKIVSA TSLLVDSILP  300
 301 GSSYEVQVRG KRLDGPGIWS DWSTPRVFTT QDVIYFPPKI LTSVGSNVSF HCIYKKENKI  360
 361 VPSKEIVWWM NLAEKIPQSQ YDVVSDHVSK VTFFNLNETK PRGKFTYDAV YCCNEHECHH  420
 421 RYAELYVIDV NINISCETDG YLTKMTCRWS TSTIQSLAES TLQLRYHRSS LYCSDIPSIH  480
 481 PISEPKDCYL QSDGFYECIF QPIFLLSGYT MWIRINHSLG SLDSPPTCVL PDSVVKPLPP  540
 541 SSVKAEITIN IGLLKISWEK PVFPENNLQF QIRYGLSGKE VQWKMYEVYD AKSKSVSLPV  600
 601 PDLCAVYAVQ VRCKRLDGLG YWSNWSNPAY TVVMDIKVPM RGPEFWRIIN GDTMKKEKNV  660
 661 TLLWKPLMKN DSLCSVQRYV INHHTSCNGT WSEDVGNHTK FTFLWTEQAH TVTVLAINSI  720
 721 GASVANFNLT FSWPMSKVNI VQSLSAYPLN SSCVIVSWIL SPSDYKLMYF IIEWKNLNED  780
 781 GEIKWLRISS SVKKYYIHDH FIPIEKYQFS LYPIFMEGVG KPKIINSFTQ DDIEKHQSDA  840
 841 GLYVIVPVII SSSILLLGTL LISHQRMKKL FWEDVPNPKN CSWAQGLNFQ KMLEGSMFVK  900
 901 SHHHSLISST QGHKHCGRPQ GPLHRKTRDL CSLVYLLTLP PLLSYDPAKS PSVRNTQE    958
```

Figur 2d:
Leptinrezeptor Isoform D (Homo sapiens)

```
   1 MICQKFCVVL LHWEFIYVIT AFNLSYPITP WRFKLSCMPP NSTYDYFLLP AGLSKNTSNS   60
  61 NGHYETAVEP KFNSSGTHFS NLSKTTFHCC FRSEQDRNCS LCADNIEGKT FVSTVNSLVF  120
 121 QQIDANWNIQ CWLKGDLKLF ICYVESLFKN LFRNYNYKVH LLYVLPEVLE DSPLVPQKGS  180
 181 FQMVHCNCSV HECCECLVPV PTAKLNDTLL MCLKITSGGV IFQSPLMSVQ PINMVKPDPP  240
 241 LGLHMEITDD GNLKISWSSP PLVPFPLQYQ VKYSENSTTV IREADKIVSA TSLLVDSILP  300
 301 GSSYEVQVRG KRLDGPGIWS DWSTPRVFTT QDVIYFPPKI LTSVGSNVSF HCIYKKENKI  360
 361 VPSKEIVWWM NLAEKIPQSQ YDVVSDHVSK VTFFNLNETK PRGKFTYDAV YCCNEHECHH  420
 421 RYAELYVIDV NINISCETDG YLTKMTCRWS TSTIQSLAES TLQLRYHRSS LYCSDIPSIH  480
 481 PISEPKDCYL QSDGFYECIF QPIFLLSGYT MWIRINHSLG SLDSPPTCVL PDSVVKPLPP  540
 541 SSVKAEITIN IGLLKISWEK PVFPENNLQF QIRYGLSGKE VQWKMYEVYD AKSKSVSLPV  600
 601 PDLCAVYAVQ VRCKRLDGLG YWSNWSNPAY TVVMDIKVPM RGPEFWRIIN GDTMKKEKNV  660
 661 TLLWKPLMKN DSLCSVQRYV INHHTSCNGT WSEDVGNHTK FTFLWTEQAH TVTVLAINSI  720
 721 GASVANFNLT FSWPMSKVNI VQSLSAYPLN SSCVIVSWIL SPSDYKLMYF IIEWKNLNED  780
 781 GEIKWLRISS SVKKYYIHDH FIPIEKYQFS LYPIFMEGVG KPKIINSFTQ DDIEKHQSDA  840
 841 GLYVIVPVII SSSILLLGTL LISHQRMKKL FWEDVPNPKN CSWAQGLNFQ KKMPGTKELL  900
 901 GGGWLT                                                            906
```

Figur 2e:
Leptinrezeptor Isoform E (Homo sapiens)

```
   1 MICQKFCVVL LHWEFIYVIT AFNLSYPITP WRFKLSCMPP NSTYDYFLLP AGLSKNTSNS   60
  61 NGHYETAVEP KFNSSGTHFS NLSKTTFHCC FRSEQDRNCS LCADNIEGKT FVSTVNSLVF  120
 121 QQIDANWNIQ CWLKGDLKLF ICYVESLFKN LFRNYNYKVH LLYVLPEVLE DSPLVPQKGS  180
 181 FQMVHCNCSV HECCECLVPV PTAKLNDTLL MCLKITSGGV IFQSPLMSVQ PINMVKPDPP  240
```

```
241 LGLHMEITDD GNLKISWSSP PLVPFPLQYQ VKYSENSTTV IREADKIVSA TSLLVDSILP 300
301 GSSYEVQVRG KRLDGPGIWS DWSTPRVFTT QDVIYFPPKI LTSVGSNVSF HCIYKKENKI 360
361 VPSKEIVWWM NLAEKIPQSQ YDVVSDHVSK VTFFNLNETK PRGKFTYDAV YCCNEHECHH 420
421 RYAELYVIDV NINISCETDG YLTKMTCRWS TSTIQSLAES TLQLRYHRSS LYCSDIPSIH 480
481 PISEPKDCYL QSDGFYECIF QPIFLLSGYT MWIRINHSLG SLDSPPTCVL PDSVVKPLPP 540
541 SSVKAEITIN IGLLKISWEK PVFPENNLQF QIRYGLSGKE VQWKMYEVYD AKSKSVSLPV 600
601 PDLCAVYAVQ VRCKRLDGLG YWSNWSNPAY TVVMDIKVPM RGPEFWRIIN GDTMKKEKNV 660
661 TLLWKPLMKN DSLCSVQRYV INHHTSCNGT WSEDVGNHTK FTFLWTEQAH TVTVLAINSI 720
721 GASVANFNLT FSWPMSKVNI VQSLSAYPLN SSCVIVSWIL SPSDYKLMYF IIEWKNLNED 780
781 GEIKWLRISS SVKKYYIHDH FIPIEKYQFS LYPIFMEGVG KPKIINSFTQ DDIEKHQSD  839
```

Figur 2f:
Leptinrezeptor Isoform B Fragment Thr20 - Asp839 (Homo sapiens)

```
  1 TAFNLSYPIT PWRFKLSCMP PNSTYDYFLL PAGLSKNTSN SNGHYETAVE PKFNSSGTHF  60
 61 SNLSKTTFHC CFRSEQDRNC SLCADNIEGK TFVSTVNSLV FQQIDANWNI QCWLKGDLKL 120
121 FICYVESLFK NLFRNYNYKV HLLYVLPEVL EDSPLVPQKG SFQMVHCNCS VHECCECLVP 180
181 VPTAKLNDTL LMCLKITSGG VIFQSPLMSV QPINMVKPDP PLGLHMEITD DGNLKISWSS 240
241 PPLVPFPLQY QVKYSENSTT VIREADKIVS ATSLLVDSIL PGSSYEVQVR GKRLDGPGIW 300
301 SDWSTPRVFT TQDVIYFPPK ILTSVGSNVS FHCIYKKENK IVPSKEIVWW MNLAEKIPQS 360
361 QYDVVSDHVS KVTFFNLNET KPRGKFTYDA VYCCNEHECH HRYAELYVID VNINISCETD 420
421 GYLTKMTCRW STSTIQSLAE STLQLRYHRS SLYCSDIPSI HPISEPKDCY LQSDGFYECI 480
481 FQPIFLLSGY TMWIRINHSL GSLDSPPTCV LPDSVVKPLP PSSVKAEITI NIGLLKISWE 540
541 KPVFPENNLQ FQIRYGLSGK EVQWKMYEVY DAKSKSVSLP VPDLCAVYAV QVRCKRLDGL 600
601 GYWSNWSNPA YTVVMDIKVP MRGPEFWRII NGDTMKKEKN VTLLWKPLMK NDSLCSVQRY 660
661 VINHHTSCNG TWSEDVGNHT KFTFLWTEQA HTVTVLAINS IGASVANFNL TFSWPMSKVN 720
721 IVQSLSAYPL NSSCVIVSWI LSPSDYKLMY FIIEWKNLNE DGEIKWLRIS SSVKKYYIHD 780
781 HFIPIEKYQF SLYPIFMEGV GKPKIINSFT QDDIEKHQSD                       820
```

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **KELESIDIS T. et al.** Narrative Review: The Role of Leptin in Human Physiology: Emerging Clinical Applications. *Ann Intern Med.,* 19. Januar 2010, vol. 152 (2), 93-100
- **PAZ-FILHO G. et al.** Leptin treatment: Facts and expectations. *Metabolism,* 2014, vol. 1-11
- **WABITSCH et al.** *N Engl J Med,* 01. Januar 2015, vol. 372 (1), 48-54
- **KRATZSCH et al.** A Rapid Quantitative Immunofunctional Assay for Measuring Human Leptin. *Horm Res,* 2002, vol. 57, 127-132
- **LAHLOU et al.** *Diabetes,* August 2000, vol. 49, 1347-1352
- **WU et al.** *J Clin Endocrinol Metab,* Juni 2002, vol. 87 (6), 2931-2939
- **POSOVSZKY et al.** *J Clin Endocrinol Metab,* Juni 2010, vol. 95 (6), 2836-2840
- **MAZEN et al.** *Molecular Genetics and Metabolism,* 2009, vol. 97, 305-308
- **FUNCKE et al.** *Molecular and Cellular Pediatrics,* 2014, vol. 1, 3
- **SAMBROOK et al.** Molecular cloning: a laboratory handbook. Corld Spring Harbor Laboratory Press, 1998